# EUROPEAN PATENT SPECIFICATION

(11) **EP 2 607 484 B1**
(45) Date of publication and mention of the grant of the patent: **06.01.2016**
(21) Application number: 13160338.3
(22) Date of filing: 13.01.2009
(51) Int. Cl.: C12N 15/113, A61K 31/7105

(54) **Methods and means for efficient skipping of exon 45 in Duchenne Muscular Dystrophy pre-mRNA**
VERFAHREN UND MITTEL ZUM WIRKSAMEN ÜBERSPRINGEN VON EXON 45 IN DUCHENNE-MUSKELDYSTROPHIE-PRÄ-MRNA
PROCÉDÉS ET MOYENS D'INDUCTION DU SAUT DE L EXON 45 DANS L ARN PRÉ-MESSAGER DU GÈNE DE LA DYSTROPHIE MUSCULAIRE DE DUCHENNE

(30) Priority: 27.10.2008 WO PCT/NL2008/050673
(43) Date of publication of application: 26.06.2013
(62) Divisional of application: 09788160.1
(73) Proprietor: BioMarin Technologies B.V., 2333 CH Leiden (NL); Academisch Ziekenhuis Leiden, 2333 ZA Leiden (NL)
(72) Inventor: de Kimpe, Josephus Johannes, 3521 BE Utrecht (NL); Platenburg, Gerardus Johannes, 2252 XR Voorschoten (NL); van Deutekom, Judith Christina Theodora, 3329 AA Dordrecht (NL); Aartsma-Rus, Annemieke, 2134 BE Hoofddorp (NL); van Ommen, Garrit-Jan Boudewijn, 1015 LW Amsterdam (NL)
(74) Representative: Nederlandsch Octrooibureau

(56) References cited:
- EP-A- 1 191 098
- WO-A-2007/135105
- WO-A1-2010/048586
- AARTSMA-RUS ET AL: "Exploring the Frontiers of Therapeutic Exon Skipping for Duchenne Muscular Dystrophy by Double Targeting within One or Multiple Exons", MOLECULAR THERAPY, ACADEMIC PRESS, SAN DIEGO, CA, US, vol. 14, no. 3, 12 August 2006 (2006-08-12), pages 401-407, XP005844833, ISSN: 1525-0016
- AARTSMA-RUS ANNEMIEKE ET AL: "Guidelines for antisense oligonucleotide design and insight into splice-modulating mechanisms", MOLECULAR THERAPY, ACADEMIC PRESS, SAN DIEGO, CA, US, vol. 17, no. 3, 23 September 2008 (2008-09-23), pages 548-553, XP008117483, ISSN: 1525-0016, DOI: 10.1038/MT.2008.205 [retrieved on 2008-09-23]
- HEEMSKERK HANS A ET AL: "In vivo comparison of 2'-O-methyl phosphorothioate and morpholino antisense oligonucleotides for Duchenne muscular dystrophy exon skipping", JOURNAL OF GENE MEDICINE, JOHN WILEY & SONS, INC, US, vol. 11, no. 3, 1 March 2009 (2009-03-01), pages 257-266, XP002541504, ISSN: 1099-498X, DOI: 10.1002/JGM.1288 [retrieved on 2009-01-12]

### SEQUENCE LISTING

<110> Academisch Ziekenhuis Leiden
   Prosensa B.V.
   Prosensa Holding B.V.
   Prosensa Technologies B.V.
<120> Methods and means for efficient skipping of exon 45 in DMD pre-mRNA
<130> P6024691PCT
<150> PCT/NL2008/50673
   <151> 2008-10-27
<160> 236
<170> PatentIn version 3.3
<210> 1
   <211> 3685
   <212> PRT
   <213> homo sapiens
<400> 1
   Met Leu Trp Trp Glu Glu Val Glu Asp Cys Tyr Glu Arg Glu Asp Val
   1               5                   10                  15
   Gln Lys Lys Thr Phe Thr Lys Trp Val Asn Ala Gln Phe Ser Lys Phe
               20                  25                  30
   Gly Lys Gln His Ile Glu Asn Leu Phe Ser Asp Leu Gln Asp Gly Arg
           35                  40                  45
   Arg Leu Leu Asp Leu Leu Glu Gly Leu Thr Gly Gln Lys Leu Pro Lys
       50                  55                  60
   Glu Lys Gly Ser Thr Arg Val His Ala Leu Asn Asn Val Asn Lys Ala
   65                  70                  75                  80
   Leu Arg Val Leu Gln Asn Asn Asn Val Asp Leu Val Asn Ile Gly Ser
                   85                  90                  95
   Thr Asp Ile Val Asp Gly Asn His Lys Leu Thr Leu Gly Leu Ile Trp
               100                 105                 110
   Asn Ile Ile Leu His Trp Gln Val Lys Asn Val Met Lys Asn Ile Met
           115                 120                 125
   Ala Gly Leu Gln Gln Thr Asn Ser Glu Lys Ile Leu Leu Ser Trp Val
       130                 135                 140
   Arg Gln Ser Thr Arg Asn Tyr Pro Gln Val Asn Val Ile Asn Phe Thr
   145                 150                 155                 160
   Thr Ser Trp Ser Asp Gly Leu Ala Leu Asn Ala Leu Ile His Ser His
                   165                 170                 175
   Arg Pro Asp Leu Phe Asp Trp Asn Ser Val Val Cys Gln Gln Ser Ala
               180                 185                 190
   Thr Gln Arg Leu Glu His Ala Phe Asn Ile Ala Arg Tyr Gln Leu Gly
           195                 200                 205
   Ile Glu Lys Leu Leu Asp Pro Glu Asp Val Asp Thr Thr Tyr Pro Asp
       210                 215                 220
   Lys Lys Ser Ile Leu Met Tyr Ile Thr Ser Leu Phe Gln Val Leu Pro
   225                 230                 235                 240
   Gln Gln Val Ser Ile Glu Ala Ile Gln Glu Val Glu Met Leu Pro Arg
                   245                 250                 255
   Pro Pro Lys Val Thr Lys Glu Glu His Phe Gln Leu His His Gln Met
               260                 265                 270
   His Tyr Ser Gln Gln Ile Thr Val Ser Leu Ala Gln Gly Tyr Glu Arg
           275                 280                 285
   Thr Ser Ser Pro Lys Pro Arg Phe Lys Ser Tyr Ala Tyr Thr Gln Ala
       290                 295                 300
   Ala Tyr Val Thr Thr Ser Asp Pro Thr Arg Ser Pro Phe Pro Ser Gln
   305                 310                 315                 320
   His Leu Glu Ala Pro Glu Asp Lys Ser Phe Gly Ser Ser Leu Met Glu
                   325                 330                 335
   Ser Glu Val Asn Leu Asp Arg Tyr Gln Thr Ala Leu Glu Glu Val Leu
               340                 345                 350
   Ser Trp Leu Leu Ser Ala Glu Asp Thr Leu Gln Ala Gln Gly Glu Ile
           355                 360                 365
   Ser Asn Asp Val Glu Val Val Lys Asp Gln Phe His Thr His Glu Gly
       370                 375                 380
   Tyr Met Met Asp Leu Thr Ala His Gln Gly Arg Val Gly Asn Ile Leu
   385                 390                 395                 400
   Gln Leu Gly Ser Lys Leu Ile Gly Thr Gly Lys Leu Ser Glu Asp Glu
                   405                 410                 415
   Glu Thr Glu Val Gln Glu Gln Met Asn Leu Leu Asn Ser Arg Trp Glu
               420                 425                 430
   Cys Leu Arg Val Ala Ser Met Glu Lys Gln Ser Asn Leu His Arg Val
           435                 440                 445
   Leu Met Asp Leu Gln Asn Gln Lys Leu Lys Glu Leu Asn Asp Trp Leu
       450                 455                 460
   Thr Lys Thr Glu Glu Arg Thr Arg Lys Met Glu Glu Glu Pro Leu Gly
   465                 470                 475                 480
   Pro Asp Leu Glu Asp Leu Lys Arg Gln Val Gln Gln His Lys Val Leu
                   485                 490                 495
   Gln Glu Asp Leu Glu Gln Glu Gln Val Arg Val Asn Ser Leu Thr His
               500                 505                 510
   Met Val Val Val Val Asp Glu Ser Ser Gly Asp His Ala Thr Ala Ala
           515                 520                 525
   Leu Glu Glu Gln Leu Lys Val Leu Gly Asp Arg Trp Ala Asn Ile Cys
       530                 535                 540
   Arg Trp Thr Glu Asp Arg Trp Val Leu Leu Gln Asp Ile Leu Leu Lys
   545                 550                 555                 560
   Trp Gln Arg Leu Thr Glu Glu Gln Cys Leu Phe Ser Ala Trp Leu Ser
                   565                 570                 575
   Glu Lys Glu Asp Ala Val Asn Lys Ile His Thr Thr Gly Phe Lys Asp
               580                 585                 590
   Gln Asn Glu Met Leu Ser Ser Leu Gln Lys Leu Ala Val Leu Lys Ala
           595                 600                 605
   Asp Leu Glu Lys Lys Lys Gln Ser Met Gly Lys Leu Tyr Ser Leu Lys
       610                 615                 620
   Gln Asp Leu Leu Ser Thr Leu Lys Asn Lys Ser Val Thr Gln Lys Thr
   625                 630                 635                 640
   Glu Ala Trp Leu Asp Asn Phe Ala Arg Cys Trp Asp Asn Leu Val Gln
                   645                 650                 655
   Lys Leu Glu Lys Ser Thr Ala Gln Ile Ser Gln Ala Val Thr Thr Thr
               660                 665                 670
   Gln Pro Ser Leu Thr Gln Thr Thr Val Met Glu Thr Val Thr Thr Val
           675                 680                 685
   Thr Thr Arg Glu Gln Ile Leu Val Lys His Ala Gln Glu Glu Leu Pro
       690                 695                 700
   Pro Pro Pro Pro Gln Lys Lys Arg Gln Ile Thr Val Asp Ser Glu Ile
   705                 710                 715                 720
   Arg Lys Arg Leu Asp Val Asp Ile Thr Glu Leu His Ser Trp Ile Thr
                   725                 730                 735
   Arg Ser Glu Ala Val Leu Gln Ser Pro Glu Phe Ala Ile Phe Arg Lys
               740                 745                 750
   Glu Gly Asn Phe Ser Asp Leu Lys Glu Lys Val Asn Ala Ile Glu Arg
           755                 760                 765
   Glu Lys Ala Glu Lys Phe Arg Lys Leu Gln Asp Ala Ser Arg Ser Ala
       770                 775                 780
   Gln Ala Leu Val Glu Gln Met Val Asn Glu Gly Val Asn Ala Asp Ser
   785                 790                 795                 800
   Ile Lys Gln Ala Ser Glu Gln Leu Asn Ser Arg Trp Ile Glu Phe Cys
                   805                 810                 815
   Gln Leu Leu Ser Glu Arg Leu Asn Trp Leu Glu Tyr Gln Asn Asn Ile
               820                 825                 830
   Ile Ala Phe Tyr Asn Gln Leu Gln Gln Leu Glu Gln Met Thr Thr Thr
           835                 840                 845
   Ala Glu Asn Trp Leu Lys Ile Gln Pro Thr Thr Pro Ser Glu Pro Thr
       850                 855                 860
   Ala Ile Lys Ser Gln Leu Lys Ile Cys Lys Asp Glu Val Asn Arg Leu
   865                 870                 875                 880
   Ser Gly Leu Gln Pro Gln Ile Glu Arg Leu Lys Ile Gln Ser Ile Ala
                   885                 890                 895
   Leu Lys Glu Lys Gly Gln Gly Pro Met Phe Leu Asp Ala Asp Phe Val
               900                 905                 910
   Ala Phe Thr Asn His Phe Lys Gln Val Phe Ser Asp Val Gln Ala Arg
           915                 920                 925
   Glu Lys Glu Leu Gln Thr Ile Phe Asp Thr Leu Pro Pro Met Arg Tyr
       930                 935                 940
   Gln Glu Thr Met Ser Ala Ile Arg Thr Trp Val Gln Gln Ser Glu Thr
   945                 950                 955                 960
   Lys Leu Ser Ile Pro Gln Leu Ser Val Thr Asp Tyr Glu Ile Met Glu
                   965                 970                 975
   Gln Arg Leu Gly Glu Leu Gln Ala Leu Gln Ser Ser Leu Gln Glu Gln
               980                 985                 990
   Gln Ser Gly Leu Tyr Tyr Leu Ser  Thr Thr Val Lys Glu  Met Ser Lys
           995                 1000                 1005
   Lys Ala  Pro Ser Glu Ile Ser  Arg Lys Tyr Gln Ser  Glu Phe Glu
       1010                 1015                 1020
   Glu Ile  Glu Gly Arg Trp Lys  Lys Leu Ser Ser Gln  Leu Val Glu
       1025                 1030                 1035
   His Cys  Gln Lys Leu Glu Glu  Gln Met Asn Lys Leu  Arg Lys Ile
       1040                 1045                 1050
   Gln Asn  His Ile Gln Thr Leu  Lys Lys Trp Met Ala  Glu Val Asp
       1055                 1060                 1065
   Val Phe  Leu Lys Glu Glu Trp  Pro Ala Leu Gly Asp  Ser Glu Ile
       1070                 1075                 1080
   Leu Lys  Lys Gln Leu Lys Gln  Cys Arg Leu Leu Val  Ser Asp Ile
       1085                 1090                 1095
   Gln Thr  Ile Gln Pro Ser Leu  Asn Ser Val Asn Glu  Gly Gly Gln
       1100                 1105                 1110
   Lys Ile  Lys Asn Glu Ala Glu  Pro Glu Phe Ala Ser  Arg Leu Glu
       1115                 1120                 1125
   Thr Glu  Leu Lys Glu Leu Asn  Thr Gln Trp Asp His  Met Cys Gln
       1130                 1135                 1140
   Gln Val  Tyr Ala Arg Lys Glu  Ala Leu Lys Gly Gly  Leu Glu Lys
   1145 1150 1155
   Thr Val Ser Leu Gln Lys Asp Leu Ser Glu Met His Glu Trp Met
   1160 1165 1170
   Thr Gln Ala Glu Glu Glu Tyr Leu Glu Arg Asp Phe Glu Tyr Lys
   1175 1180 1185
   Thr Pro Asp Glu Leu Gln Lys Ala Val Glu Glu Met Lys Arg Ala
   1190 1195 1200
   Lys Glu Glu Ala Gln Gln Lys Glu Ala Lys Val Lys Leu Leu Thr
   1205 1210 1215
   Glu Ser Val Asn Ser Val Ile Ala Gln Ala Pro Pro Val Ala Gln
   1220 1225 1230
   Glu Ala Leu Lys Lys Glu Leu Glu Thr Leu Thr Thr Asn Tyr Gln
   1235 1240 1245
   Trp Leu Cys Thr Arg Leu Asn Gly Lys Cys Lys Thr Leu Glu Glu
   1250 1255 1260
   Val Trp Ala Cys Trp His Glu Leu Leu Ser Tyr Leu Glu Lys Ala
   1265 1270 1275
   Asn Lys Trp Leu Asn Glu Val Glu Phe Lys Leu Lys Thr Thr Glu
   1280 1285 1290
   Asn Ile Pro Gly Gly Ala Glu Glu Ile Ser Glu Val Leu Asp Ser
   1295 1300 1305
   Leu Glu Asn Leu Met Arg His Ser Glu Asp Asn Pro Asn Gln Ile
   1310 1315 1320
   Arg Ile Leu Ala Gln Thr Leu Thr Asp Gly Gly Val Met Asp Glu
   1325 1330 1335
   Leu Ile Asn Glu Glu Leu Glu Thr Phe Asn Ser Arg Trp Arg Glu
   1340 1345 1350
   Leu His Glu Glu Ala Val Arg Arg Gln Lys Leu Leu Glu Gln Ser
   1355 1360 1365
   Ile Gln Ser Ala Gln Glu Thr Glu Lys Ser Leu His Leu Ile Gln
   1370 1375 1380
   Glu Ser  Leu Thr Phe Ile Asp  Lys Gln Leu Ala Ala  Tyr Ile Ala
       1385                 1390                 1395
   Asp Lys  Val Asp Ala Ala Gln  Met Pro Gln Glu Ala  Gln Lys Ile
       1400                 1405                 1410
   Gln Ser  Asp Leu Thr Ser His  Glu Ile Ser Leu Glu  Glu Met Lys
       1415                 1420                 1425
   Lys His  Asn Gln Gly Lys Glu  Ala Ala Gln Arg Val  Leu Ser Gln
       1430                 1435                 1440
   Ile Asp  Val Ala Gln Lys Lys  Leu Gln Asp Val Ser  Met Lys Phe
       1445                 1450                 1455
   Arg Leu  Phe Gln Lys Pro Ala  Asn Phe Glu Gln Arg  Leu Gln Glu
       1460                 1465                 1470
   Ser Lys  Met Ile Leu Asp Glu  Val Lys Met His Leu  Pro Ala Leu
       1475                 1480                 1485
   Glu Thr  Lys Ser Val Glu Gln  Glu Val Val Gln Ser  Gln Leu Asn
       1490                 1495                 1500
   His Cys  Val Asn Leu Tyr Lys  Ser Leu Ser Glu Val  Lys Ser Glu
       1505                 1510                 1515
   Val Glu  Met Val Ile Lys Thr  Gly Arg Gln Ile Val  Gln Lys Lys
       1520                 1525                 1530
   Gln Thr  Glu Asn Pro Lys Glu  Leu Asp Glu Arg Val  Thr Ala Leu
       1535                 1540                 1545
   Lys Leu  His Tyr Asn Glu Leu  Gly Ala Lys Val Thr  Glu Arg Lys
       1550                 1555                 1560
   Gln Gln  Leu Glu Lys Cys Leu  Lys Leu Ser Arg Lys  Met Arg Lys
       1565                 1570                 1575
   Glu Met  Asn Val Leu Thr Glu  Trp Leu Ala Ala Thr  Asp Met Glu
       1580                 1585                 1590
   Leu Thr  Lys Arg Ser Ala Val  Glu Gly Met Pro Ser  Asn Leu Asp
       1595                 1600                 1605
   Ser Glu  Val Ala Trp Gly Lys  Ala Thr Gln Lys Glu  Ile Glu Lys
       1610                 1615                 1620
   Gln Lys  Val His Leu Lys Ser  Ile Thr Glu Val Gly  Glu Ala Leu
       1625                 1630                 1635
   Lys Thr  Val Leu Gly Lys Lys  Glu Thr Leu Val Glu  Asp Lys Leu
       1640                 1645                 1650
   Ser Leu  Leu Asn Ser Asn Trp  Ile Ala Val Thr Ser  Arg Ala Glu
       1655                 1660                 1665
   Glu Trp  Leu Asn Leu Leu Leu  Glu Tyr Gln Lys His  Met Glu Thr
       1670                 1675                 1680
   Phe Asp  Gln Asn Val Asp His  Ile Thr Lys Trp Ile  Ile Gln Ala
       1685                 1690                 1695
   Asp Thr  Leu Leu Asp Glu Ser  Glu Lys Lys Lys Pro  Gln Gln Lys
       1700                 1705                 1710
   Glu Asp  Val Leu Lys Arg Leu  Lys Ala Glu Leu Asn  Asp Ile Arg
       1715                 1720                 1725
   Pro Lys  Val Asp Ser Thr Arg  Asp Gln Ala Ala Asn  Leu Met Ala
       1730                 1735                 1740
   Asn Arg  Gly Asp His Cys Arg  Lys Leu Val Glu Pro  Gln Ile Ser
       1745                 1750                 1755
   Glu Leu  Asn His Arg Phe Ala  Ala Ile Ser His Arg  Ile Lys Thr
       1760                 1765                 1770
   Gly Lys  Ala Ser Ile Pro Leu  Lys Glu Leu Glu Gln  Phe Asn Ser
       1775                 1780                 1785
   Asp Ile  Gln Lys Leu Leu Glu  Pro Leu Glu Ala Glu  Ile Gln Gln
       1790                 1795                 1800
   Gly Val  Asn Leu Lys Glu Glu  Asp Phe Asn Lys Asp  Met Asn Glu
       1805                 1810                 1815
   Asp Asn  Glu Gly Thr Val Lys  Glu Leu Leu Gln Arg  Gly Asp Asn
       1820                 1825                 1830
   Leu Gln  Gln Arg Ile Thr Asp  Glu Arg Lys Arg Glu  Glu Ile Lys
       1835                 1840                 1845
   Ile Lys  Gln Gln Leu Leu Gln  Thr Lys His Asn Ala  Leu Lys Asp
       1850                 1855                 1860
   Leu Arg Ser Gln Arg Arg Lys Lys Ala Leu Glu Ile Ser His Gln
   1865 1870 1875
   Trp Tyr Gln Tyr Lys Arg Gln Ala Asp Asp Leu Leu Lys Cys Leu
   1880 1885 1890
   Asp Asp Ile Glu Lys Lys Leu Ala Ser Leu Pro Glu Pro Arg Asp
   1895 1900 1905
   Glu Arg Lys Ile Lys Glu Ile Asp Arg Glu Leu Gln Lys Lys Lys
   1910 1915 1920
   Glu Glu Leu Asn Ala Val Arg Arg Gln Ala Glu Gly Leu Ser Glu
   1925 1930 1935
   Asp Gly Ala Ala Met Ala Val Glu Pro Thr Gln Ile Gln Leu Ser
   1940 1945 1950
   Lys Arg Trp Arg Glu Ile Glu Ser Lys Phe Ala Gln Phe Arg Arg
   1955 1960 1965
   Leu Asn Phe Ala Gln Ile His Thr Val Arg Glu Glu Thr Met Met
   1970 1975 1980
   Val Met Thr Glu Asp Met Pro Leu Glu Ile Ser Tyr Val Pro Ser
   1985 1990 1995
   Thr Tyr Leu Thr Glu Ile Thr His Val Ser Gln Ala Leu Leu Glu
   2000 2005 2010
   Val Glu Gln Leu Leu Asn Ala Pro Asp Leu Cys Ala Lys Asp Phe
   2015 2020 2025
   Glu Asp Leu Phe Lys Gln Glu Glu Ser Leu Lys Asn Ile Lys Asp
   2030 2035 2040
   Ser Leu Gln Gln Ser Ser Gly Arg Ile Asp Ile Ile His Ser Lys
   2045 2050 2055
   Lys Thr Ala Ala Leu Gln Ser Ala Thr Pro Val Glu Arg Val Lys
   2060 2065 2070
   Leu Gln Glu Ala Leu Ser Gln Leu Asp Phe Gln Trp Glu Lys Val
   2075 2080 2085
   Asn Lys Met Tyr Lys Asp Arg Gln Gly Arg Phe Asp Arg Ser Val
   2090 2095 2100
   Glu Lys Trp Arg Arg Phe His Tyr Asp Ile Lys Ile Phe Asn Gln
   2105 2110 2115
   Trp Leu Thr Glu Ala Glu Gln Phe Leu Arg Lys Thr Gln Ile Pro
   2120 2125 2130
   Glu Asn Trp Glu His Ala Lys Tyr Lys Trp Tyr Leu Lys Glu Leu
   2135 2140 2145
   Gln Asp Gly Ile Gly Gln Arg Gln Thr Val Val Arg Thr Leu Asn
   2150 2155 2160
   Ala Thr Gly Glu Glu Ile Ile Gln Gln Ser Ser Lys Thr Asp Ala
   2165 2170 2175
   Ser Ile Leu Gln Glu Lys Leu Gly Ser Leu Asn Leu Arg Trp Gln
   2180 2185 2190
   Glu Val Cys Lys Gln Leu Ser Asp Arg Lys Lys Arg Leu Glu Glu
   2195 2200 2205
   Gln Lys Asn Ile Leu Ser Glu Phe Gln Arg Asp Leu Asn Glu Phe
   2210 2215 2220
   Val Leu Trp Leu Glu Glu Ala Asp Asn Ile Ala Ser Ile Pro Leu
   2225 2230 2235
   Glu Pro Gly Lys Glu Gln Gln Leu Lys Glu Lys Leu Glu Gln Val
   2240 2245 2250
   Lys Leu Leu Val Glu Glu Leu Pro Leu Arg Gln Gly Ile Leu Lys
   2255 2260 2265
   Gln Leu Asn Glu Thr Gly Gly Pro Val Leu Val Ser Ala Pro Ile
   2270 2275 2280
   Ser Pro Glu Glu Gln Asp Lys Leu Glu Asn Lys Leu Lys Gln Thr
   2285 2290 2295
   Asn Leu Gln Trp Ile Lys Val Ser Arg Ala Leu Pro Glu Lys Gln
   2300 2305 2310
   Gly Glu Ile Glu Ala Gln Ile Lys Asp Leu Gly Gln Leu Glu Lys
   2315 2320 2325
   Lys Leu  Glu Asp Leu Glu Glu  Gln Leu Asn His Leu  Leu Leu Trp
       2330                 2335                 2340
   Leu Ser  Pro Ile Arg Asn Gln  Leu Glu Ile Tyr Asn  Gln Pro Asn
       2345                 2350                 2355
   Gln Glu  Gly Pro Phe Asp Val  Gln Glu Thr Glu Ile  Ala Val Gln
       2360                 2365                 2370
   Ala Lys  Gln Pro Asp Val Glu  Glu Ile Leu Ser Lys  Gly Gln His
       2375                 2380                 2385
   Leu Tyr  Lys Glu Lys Pro Ala  Thr Gln Pro Val Lys  Arg Lys Leu
       2390                 2395                 2400
   Glu Asp  Leu Ser Ser Glu Trp  Lys Ala Val Asn Arg  Leu Leu Gln
       2405                 2410                 2415
   Glu Leu  Arg Ala Lys Gln Pro  Asp Leu Ala Pro Gly  Leu Thr Thr
       2420                 2425                 2430
   Ile Gly  Ala Ser Pro Thr Gln  Thr Val Thr Leu Val  Thr Gln Pro
       2435                 2440                 2445
   Val Val  Thr Lys Glu Thr Ala  Ile Ser Lys Leu Glu  Met Pro Ser
       2450                 2455                 2460
   Ser Leu  Met Leu Glu Val Pro  Ala Leu Ala Asp Phe  Asn Arg Ala
       2465                 2470                 2475
   Trp Thr  Glu Leu Thr Asp Trp  Leu Ser Leu Leu Asp  Gln Val Ile
       2480                 2485                 2490
   Lys Ser  Gln Arg Val Met Val  Gly Asp Leu Glu Asp  Ile Asn Glu
       2495                 2500                 2505
   Met Ile  Ile Lys Gln Lys Ala  Thr Met Gln Asp Leu  Glu Gln Arg
       2510                 2515                 2520
   Arg Pro  Gln Leu Glu Glu Leu  Ile Thr Ala Ala Gln  Asn Leu Lys
       2525                 2530                 2535
   Asn Lys  Thr Ser Asn Gln Glu  Ala Arg Thr Ile Ile  Thr Asp Arg
       2540                 2545                 2550
   Ile Glu  Arg Ile Gln Asn Gln  Trp Asp Glu Val Gln  Glu His Leu
       2555                 2560                 2565
   Gln Asn Arg Arg Gln Gln Leu Asn Glu Met Leu Lys Asp Ser Thr
   2570 2575 2580
   Gln Trp Leu Glu Ala Lys Glu Glu Ala Glu Gln Val Leu Gly Gln
   2585 2590 2595
   Ala Arg Ala Lys Leu Glu Ser Trp Lys Glu Gly Pro Tyr Thr Val
   2600 2605 2610
   Asp Ala Ile Gln Lys Lys Ile Thr Glu Thr Lys Gln Leu Ala Lys
   2615 2620 2625
   Asp Leu Arg Gln Trp Gln Thr Asn Val Asp Val Ala Asn Asp Leu
   2630 2635 2640
   Ala Leu Lys Leu Leu Arg Asp Tyr Ser Ala Asp Asp Thr Arg Lys
   2645 2650 2655
   Val His Met Ile Thr Glu Asn Ile Asn Ala Ser Trp Arg Ser Ile
   2660 2665 2670
   His Lys Arg Val Ser Glu Arg Glu Ala Ala Leu Glu Glu Thr His
   2675 2680 2685
   Arg Leu Leu Gln Gln Phe Pro Leu Asp Leu Glu Lys Phe Leu Ala
   2690 2695 2700
   Trp Leu Thr Glu Ala Glu Thr Thr Ala Asn Val Leu Gln Asp Ala
   2705 2710 2715
   Thr Arg Lys Glu Arg Leu Leu Glu Asp Ser Lys Gly Val Lys Glu
   2720 2725 2730
   Leu Met Lys Gln Trp Gln Asp Leu Gln Gly Glu Ile Glu Ala His
   2735 2740 2745
   Thr Asp Val Tyr His Asn Leu Asp Glu Asn Ser Gln Lys Ile Leu
   2750 2755 2760
   Arg Ser Leu Glu Gly Ser Asp Asp Ala Val Leu Leu Gln Arg Arg
   2765 2770 2775
   Leu Asp Asn Met Asn Phe Lys Trp Ser Glu Leu Arg Lys Lys Ser
   2780 2785 2790
   Leu Asn Ile Arg Ser His Leu Glu Ala Ser Ser Asp Gln Trp Lys
   2795 2800 2805
   Arg Leu His Leu Ser Leu Gln Glu Leu Leu Val Trp Leu Gln Leu
       2810                2815                2820
   Lys Asp Asp Glu Leu Ser Arg Gln Ala Pro Ile Gly Gly Asp Phe
       2825                2830                2835
   Pro Ala Val Gln Lys Gln Asn Asp Val His Arg Ala Phe Lys Arg
       2840                2845                2850
   Glu Leu Lys Thr Lys Glu Pro Val Ile Met Ser Thr Leu Glu Thr
       2855                2860                2865
   Val Arg Ile Phe Leu Thr Glu Gln Pro Leu Glu Gly Leu Glu Lys
       2870                2875                2880
   Leu Tyr Gln Glu Pro Arg Glu Leu Pro Pro Glu Glu Arg Ala Gln
       2885                2890                2895
   Asn Val Thr Arg Leu Leu Arg Lys Gln Ala Glu Glu Val Asn Thr
       2900                2905                2910
   Glu Trp Glu Lys Leu Asn Leu His Ser Ala Asp Trp Gln Arg Lys
       2915                2920                2925
   Ile Asp Glu Thr Leu Glu Arg Leu Gln Glu Leu Gln Glu Ala Thr
       2930                2935                2940
   Asp Glu Leu Asp Leu Lys Leu Arg Gln Ala Glu Val Ile Lys Gly
       2945                2950                2955
   Ser Trp Gln Pro Val Gly Asp Leu Leu Ile Asp Ser Leu Gln Asp
       2960                2965                2970
   His Leu Glu Lys Val Lys Ala Leu Arg Gly Glu Ile Ala Pro Leu
       2975                2980                2985
   Lys Glu Asn Val Ser His Val Asn Asp Leu Ala Arg Gln Leu Thr
       2990                2995                3000
   Thr Leu Gly Ile Gln Leu Ser Pro Tyr Asn Leu Ser Thr Leu Glu
       3005                3010                3015
   Asp Leu Asn Thr Arg Trp Lys Leu Leu Gln Val Ala Val Glu Asp
       3020                3025                3030
   Arg Val Arg Gln Leu His Glu Ala His Arg Asp Phe Gly Pro Ala
   3035 3040 3045
   Ser Gln His Phe Leu Ser Thr Ser Val Gln Gly Pro Trp Glu Arg
   3050 3055 3060
   Ala Ile Ser Pro Asn Lys Val Pro Tyr Tyr Ile Asn His Glu Thr
   3065 3070 3075
   Gln Thr Thr Cys Trp Asp His Pro Lys Met Thr Glu Leu Tyr Gln
   3080 3085 3090
   Ser Leu Ala Asp Leu Asn Asn Val Arg Phe Ser Ala Tyr Arg Thr
   3095 3100 3105
   Ala Met Lys Leu Arg Arg Leu Gln Lys Ala Leu Cys Leu Asp Leu
   3110 3115 3120
   Leu Ser Leu Ser Ala Ala Cys Asp Ala Leu Asp Gln His Asn Leu
   3125 3130 3135
   Lys Gln Asn Asp Gln Pro Met Asp Ile Leu Gln Ile Ile Asn Cys
   3140 3145 3150
   Leu Thr Thr Ile Tyr Asp Arg Leu Glu Gln Glu His Asn Asn Leu
   3155 3160 3165
   Val Asn Val Pro Leu Cys Val Asp Met Cys Leu Asn Trp Leu Leu
   3170 3175 3180
   Asn Val Tyr Asp Thr Gly Arg Thr Gly Arg Ile Arg Val Leu Ser
   3185 3190 3195
   Phe Lys Thr Gly Ile Ile Ser Leu Cys Lys Ala His Leu Glu Asp
   3200 3205 3210
   Lys Tyr Arg Tyr Leu Phe Lys Gln Val Ala Ser Ser Thr Gly Phe
   3215 3220 3225
   Cys Asp Gln Arg Arg Leu Gly Leu Leu Leu His Asp Ser Ile Gln
   3230 3235 3240
   Ile Pro Arg Gln Leu Gly Glu Val Ala Ser Phe Gly Gly Ser Asn
   3245 3250 3255
   Ile Glu Pro Ser Val Arg Ser Cys Phe Gln Phe Ala Asn Asn Lys
   3260 3265 3270
   Pro Glu Ile Glu Ala Ala Leu Phe Leu Asp Trp Met Arg Leu Glu
   3275 3280 3285
   Pro Gln Ser Met Val Trp Leu Pro Val Leu His Arg Val Ala Ala
   3290 3295 3300
   Ala Glu Thr Ala Lys His Gln Ala Lys Cys Asn Ile Cys Lys Glu
   3305 3310 3315
   Cys Pro Ile Ile Gly Phe Arg Tyr Arg Ser Leu Lys His Phe Asn
   3320 3325 3330
   Tyr Asp Ile Cys Gln Ser Cys Phe Phe Ser Gly Arg Val Ala Lys
   3335 3340 3345
   Gly His Lys Met His Tyr Pro Met Val Glu Tyr Cys Thr Pro Thr
   3350 3355 3360
   Thr Ser Gly Glu Asp Val Arg Asp Phe Ala Lys Val Leu Lys Asn
   3365 3370 3375
   Lys Phe Arg Thr Lys Arg Tyr Phe Ala Lys His Pro Arg Met Gly
   3380 3385 3390
   Tyr Leu Pro Val Gln Thr Val Leu Glu Gly Asp Asn Met Glu Thr
   3395 3400 3405
   Pro Val Thr Leu Ile Asn Phe Trp Pro Val Asp Ser Ala Pro Ala
   3410 3415 3420
   Ser Ser Pro Gln Leu Ser His Asp Asp Thr His Ser Arg Ile Glu
   3425 3430 3435
   His Tyr Ala Ser Arg Leu Ala Glu Met Glu Asn Ser Asn Gly Ser
   3440 3445 3450
   Tyr Leu Asn Asp Ser Ile Ser Pro Asn Glu Ser Ile Asp Asp Glu
   3455 3460 3465
   His Leu Leu Ile Gln His Tyr Cys Gln Ser Leu Asn Gln Asp Ser
   3470 3475 3480
   Pro Leu Ser Gln Pro Arg Ser Pro Ala Gln Ile Leu Ile Ser Leu
   3485 3490 3495
   Glu Ser Glu Glu Arg Gly Glu Leu Glu Arg Ile Leu Ala Asp Leu
   3500 3505 3510
   Glu Glu  Glu Asn Arg Asn Leu  Gln Ala Glu Tyr Asp  Arg Leu Lys
       3515                 3520                 3525
   Gln Gln  His Glu His Lys Gly  Leu Ser Pro Leu Pro  Ser Pro Pro
       3530                 3535                 3540
   Glu Met  Met Pro Thr Ser Pro  Gln Ser Pro Arg Asp  Ala Glu Leu
       3545                 3550                 3555
   Ile Ala  Glu Ala Lys Leu Leu  Arg Gln His Lys Gly  Arg Leu Glu
       3560                 3565                 3570
   Ala Arg  Met Gln Ile Leu Glu  Asp His Asn Lys Gln  Leu Glu Ser
       3575                 3580                 3585
   Gln Leu  His Arg Leu Arg Gln  Leu Leu Glu Gln Pro  Gln Ala Glu
       3590                 3595                 3600
   Ala Lys  Val Asn Gly Thr Thr  Val Ser Ser Pro Ser  Thr Ser Leu
       3605                 3610                 3615
   Gln Arg  Ser Asp Ser Ser Gln  Pro Met Leu Leu Arg  Val Val Gly
       3620                 3625                 3630
   Ser Gln  Thr Ser Asp Ser Met  Gly Glu Glu Asp Leu  Leu Ser Pro
       3635                 3640                 3645
   Pro Gln  Asp Thr Ser Thr Gly  Leu Glu Glu Val Met  Glu Gln Leu
       3650                 3655                 3660
   Asn Asn  Ser Phe Pro Ser Ser  Arg Gly Arg Asn Thr  Pro Gly Lys
       3665                 3670                 3675
   Pro Met  Arg Glu Asp Thr Met
       3680                 3685
<210> 2
   <211> 75
   <212> RNA
   <213> artificial
<220>
   <223> exon
<400> 2
   ccaggauggc auugggcagc ggcaaacugu ugucagaaca uugaaugcaa cuggggaaga      60
   aauaauucag caauc                                                       75
<210> 3
   <211> 25
   <212> RNA
   <213> artificial
<220>
   <223> oligonucleotide
<400> 3
   uuugccgcug cccaaugcca uccug 25
<210> 4
   <211> 25
   <212> RNA
   <213> artificial
<220>
   <223> oligonucleotide
<400> 4
   auucaauguu cugacaacag uuugc 25
<210> 5
   <211> 25
   <212> RNA
   <213> artificial
<220>
   <223> oligonucleotide
<400> 5
   ccaguugcau ucaauguucu gacaa 25
<210> 6
   <211> 22
   <212> RNA
   <213> artificial
<220>
   <223> oligonucleotide
<400> 6
   caguugcauu caauguucug ac 22
<210> 7
   <211> 20
   <212> RNA
   <213> artificial
<220>
   <223> oligonucleotide
<400> 7
   aguugcauuc aauguucuga 20
<210> 8
   <211> 21
   <212> RNA
   <213> artificial
<220>
   <223> oligonucleotide
<400> 8
   gauugcugaa uuauuucuuc c 21
<210> 9
   <211> 25
   <212> RNA
   <213> artificial
<220>
   <223> oligonucleotide
<400> 9
   gauugcugaa uuauuucuuc cccag 25
<210> 10
   <211> 25
   <212> RNA
   <213> artificial
<220>
   <223> oligonucleotide
<400> 10
   auugcugaau uauuucuucc ccagu 25
<210> 11
   <211> 25
   <212> RNA
   <213> artificial
<220>
   <223> oligonucleotide
<400> 11
   uugcugaauu auuucuuccc caguu 25
<210> 12
   <211> 25
   <212> RNA
   <213> artificial
<220>
   <223> oligonucleotide
<400> 12
   ugcugaauua uuucuucccc aguug 25
<210> 13
   <211> 25
   <212> RNA
   <213> artificial
<220>
   <223> oligonucleotide
<400> 13
   gcugaauuau uucuucccca guugc 25
<210> 14
   <211> 25
   <212> RNA
   <213> artificial
<220>
   <223> oligonucleotide
<400> 14
   cugaauuauu ucuuccccag uugca 25
<210> 15
   <211> 25
   <212> RNA
   <213> artificial
<220>
   <223> oligonucleotide
<400> 15
   ugaauuauuu cuuccccagu ugcau 25
<210> 16
   <211> 25
   <212> RNA
   <213> artificial
<220>
   <223> oligonucleotide
<400> 16
   gaauuauuuc uuccccaguu gcauu 25
<210> 17
   <211> 25
   <212> RNA
   <213> artificial
<220>
   <223> oligonucleotide
<400> 17
   aauuauuucu uccccaguug cauuc 25
<210> 18
   <211> 25
   <212> RNA
   <213> artificial
<220>
   <223> oligonucleotide
<400> 18
   auuauuucuu ccccaguugc auuca 25
<210> 19
   <211> 25
   <212> RNA
   <213> artificial
<220>
   <223> oligonucleotide
<400> 19
   uuauuucuuc cccaguugca uucaa 25
<210> 20
   <211> 25
   <212> RNA
   <213> artificial
<220>
   <223> oligonucleotide
<400> 20
   uauuucuucc ccaguugcau ucaau 25
<210> 21
   <211> 25
   <212> RNA
   <213> artificial
<220>
   <223> oligonucleotide
<400> 21
   auuucuuccc caguugcauu caaug 25
<210> 22
   <211> 25
   <212> RNA
   <213> artificial
<220>
   <223> oligonucleotide
<400> 22
   uuucuucccc aguugcauuc aaugu 25
<210> 23
   <211> 25
   <212> RNA
   <213> artificial
<220>
   <223> oligonucleotide
<400> 23
   uucuucccca guugcauuca auguu 25
<210> 24
   <211> 25
   <212> RNA
   <213> artificial
<220>
   <223> oligonucleotide
<400> 24
   ucuuccccag uugcauucaa uguuc 25
<210> 25
   <211> 25
   <212> RNA
   <213> artificial
<220>
   <223> oligonucleotide
<400> 25
   cuuccccagu ugcauucaau guucu 25
<210> 26
   <211> 25
   <212> RNA
   <213> artificial
<220>
   <223> oligonucleotide
<400> 26
   uuccccaguu gcauucaaug uucug 25
<210> 27
   <211> 25
   <212> RNA
   <213> artificial
<220>
   <223> oligonucleotide
<400> 27
   uccccaguug cauucaaugu ucuga 25
<210> 28
   <211> 25
   <212> RNA
   <213> artificial
<220>
   <223> oligonucleotide
<400> 28
   ccccaguugc auucaauguu cugac 25
<210> 29
   <211> 25
   <212> RNA
   <213> artificial
<220>
   <223> oligonucleotide
<400> 29
   cccaguugca uucaauguuc ugaca 25
<210> 30
   <211> 25
   <212> RNA
   <213> artificial
<220>
   <223> oligonucleotide
<400> 30
   ccaguugcau ucaauguucu gacaa 25
<210> 31
   <211> 25
   <212> RNA
   <213> artificial
<220>
   <223> oligonucleotide
<400> 31
   caguugcauu caauguucug acaac 25
<210> 32
   <211> 25
   <212> RNA
   <213> artificial
<220>
   <223> oligonucleotide
<400> 32
   aguugcauuc aauguucuga caaca 25
<210> 33
   <211> 20
   <212> RNA
   <213> artificial
<220>
   <223> oligonucleotide
<400> 33
   uccuguagaa uacuggcauc 20
<210> 34
   <211> 27
   <212> RNA
   <213> artificial
<220>
   <223> oligonucleotide
<400> 34
   ugcagaccuc cugccaccgc agauuca 27
<210> 35
   <211> 34
   <212> RNA
   <213> artificial
<220>
   <223> oligonucleotide
<400> 35
   uugcagaccu ccugccaccg cagauucagg cuuc 34
<210> 36
   <211> 25
   <212> RNA
   <213> artificial
<220>
   <223> oligonucleotide
<400> 36
   guugcauuca auguucugac aacag 25
<210> 37
   <211> 25
   <212> RNA
   <213> artificial
<220>
   <223> oligonucleotide
<400> 37
   uugcauucaa uguucugaca acagu 25
<210> 38
   <211> 25
   <212> RNA
   <213> artificial
<220>
   <223> oligonucleotide
<400> 38
   ugcauucaau guucugacaa caguu 25
<210> 39
   <211> 25
   <212> RNA
   <213> artificial
<220>
   <223> oligonucleotide
<400> 39
   gcauucaaug uucugacaac aguuu 25
<210> 40
   <211> 25
   <212> RNA
   <213> artificial
<220>
   <223> oligonucleotide
<400> 40
   cauucaaugu ucugacaaca guuug 25
<210> 41
   <211> 25
   <212> RNA
   <213> artificial
<220>
   <223> oligonucleotide
<400> 41
   auucaauguu cugacaacag uuugc 25
<210> 42
   <211> 25
   <212> RNA
   <213> artificial
<220>
   <223> oligonucleotide
<400> 42
   ucaauguucu gacaacaguu ugccg 25
<210> 43
   <211> 25
   <212> RNA
   <213> artificial
<220>
   <223> oligonucleotide
<400> 43
   caauguucug acaacaguuu gccgc 25
<210> 44
   <211> 25
   <212> RNA
   <213> artificial
<220>
   <223> oligonucleotide
<400> 44
   aauguucuga caacaguuug ccgcu 25
<210> 45
   <211> 25
   <212> RNA
   <213> artificial
<220>
   <223> oligonucleotide
<400> 45
   auguucugac aacaguuugc cgcug 25
<210> 46
   <211> 25
   <212> RNA
   <213> artificial
<220>
   <223> oligonucleotide
<400> 46
   uguucugaca acaguuugcc gcugc 25
<210> 47
   <211> 25
   <212> RNA
   <213> artificial
<220>
   <223> oligonucleotide
<400> 47
   guucugacaa caguuugccg cugcc 25
<210> 48
   <211> 25
   <212> RNA
   <213> artificial
<220>
   <223> oligonucleotide
<400> 48
   uucugacaac aguuugccgc ugccc 25
<210> 49
   <211> 25
   <212> RNA
   <213> artificial
<220>
   <223> oligonucleotide
<400> 49
   ucugacaaca guuugccgcu gccca 25
<210> 50
   <211> 25
   <212> RNA
   <213> artificial
<220>
   <223> oligonucleotide
<400> 50
   cugacaacag uuugccgcug cccaa 25
<210> 51
   <211> 25
   <212> RNA
   <213> artificial
<220>
   <223> oligonucleotide
<400> 51
   ugacaacagu uugccgcugc ccaau 25
<210> 52
   <211> 25
   <212> RNA
   <213> artificial
<220>
   <223> oligonucleotide
<400> 52
   gacaacaguu ugccgcugcc caaug 25
<210> 53
   <211> 25
   <212> RNA
   <213> artificial
<220>
   <223> oligonucleotide
<400> 53
   acaacaguuu gccgcugccc aaugc 25
<210> 54
   <211> 25
   <212> RNA
   <213> artificial
<220>
   <223> oligonucleotide
<400> 54
   caacaguuug ccgcugccca augcc 25
<210> 55
   <211> 25
   <212> RNA
   <213> artificial
<220>
   <223> oligonucleotide
<400> 55
   aacaguuugc cgcugcccaa ugcca 25
<210> 56
   <211> 25
   <212> RNA
   <213> artificial
<220>
   <223> oligonucleotide
<400> 56
   acaguuugcc gcugcccaau gccau 25
<210> 57
   <211> 25
   <212> RNA
   <213> artificial
<220>
   <223> oligonucleotide
<400> 57
   caguuugccg cugcccaaug ccauc 25
<210> 58
   <211> 25
   <212> RNA
   <213> artificial
<220>
   <223> oligonucleotide
<400> 58
   aguuugccgc ugcccaaugc caucc 25
<210> 59
   <211> 25
   <212> RNA
   <213> artificial
<220>
   <223> oligonucleotide
<400> 59
   guuugccgcu gcccaaugcc auccu 25
<210> 60
   <211> 25
   <212> RNA
   <213> artificial
<220>
   <223> oligonucleotide
<400> 60
   uuugccgcug cccaaugcca uccug 25
<210> 61
   <211> 25
   <212> RNA
   <213> artificial
<220>
   <223> oligonucleotide
<400> 61
   uugccgcugc ccaaugccau ccugg 25
<210> 62
   <211> 25
   <212> RNA
   <213> artificial
<220>
   <223> oligonucleotide
<400> 62
   ugccgcugcc caaugccauc cugga 25
<210> 63
   <211> 25
   <212> RNA
   <213> artificial
<220>
   <223> oligonucleotide
<400> 63
   gccgcugccc aaugccaucc uggag 25
<210> 64
   <211> 25
   <212> RNA
   <213> artificial
<220>
   <223> oligonucleotide
<400> 64
   ccgcugccca augccauccu ggagu 25
<210> 65
   <211> 25
   <212> RNA
   <213> artificial
<220>
   <223> oligonucleotide
<400> 65
   cgcugcccaa ugccauccug gaguu 25
<210> 66
   <211> 20
   <212> RNA
   <213> artificial
<220>
   <223> oligoncleotide
<400> 66
   uguuuuugag gauugcugaa 20
<210> 67
   <211> 40
   <212> RNA
   <213> artificial
<220>
   <223> oligonucleotide
<400> 67
   uguucugaca acaguuugcc gcugcccaau gccauccugg 40
<210> 68
   <211> 17
   <212> RNA
   <213> artificial
<220>
   <223> oligonucleotide
<400> 68
   gcccaaugcc auccugg 17
<210> 69
   <211> 25
   <212> RNA
   <213> artificial
<220>
   <223> oligonucleotide
<400> 69
   agagcaggua ccuccaacau caagg 25
<210> 70
   <211> 25
   <212> RNA
   <213> artificial
<220>
   <223> oligonucleotide
<400> 70
   gagcagguac cuccaacauc aagga 25
<210> 71
   <211> 25
   <212> RNA
   <213> artificial
<220>
   <223> oligonucleotide
<400> 71
   agcagguacc uccaacauca aggaa 25
<210> 72
   <211> 25
   <212> RNA
   <213> artificial
<220>
   <223> oligonucleotide
<400> 72
   gcagguaccu ccaacaucaa ggaag 25
<210> 73
   <211> 25
   <212> RNA
   <213> artificial
<220>
   <223> oligonucleotide
<400> 73
   cagguaccuc caacaucaag gaaga 25
<210> 74
   <211> 25
   <212> RNA
   <213> artificial
<220>
   <223> oligonucleotide
<400> 74
   agguaccucc aacaucaagg aagau 25
<210> 75
   <211> 25
   <212> RNA
   <213> artificial
<220>
   <223> oligonucleotide
<400> 75
   gguaccucca acaucaagga agaug 25
<210> 76
   <211> 25
   <212> RNA
   <213> artificial
<220>
   <223> oligonucleotide
<400> 76
   guaccuccaa caucaaggaa gaugg 25
<210> 77
   <211> 25
   <212> RNA
   <213> artificial
<220>
   <223> oligonucleotide
<400> 77
   uaccuccaac aucaaggaag auggc 25
<210> 78
   <211> 25
   <212> RNA
   <213> artificial
<220>
   <223> oligonucleotide
<400> 78
   accuccaaca ucaaggaaga uggca 25
<210> 79
   <211> 25
   <212> RNA
   <213> artificial
<220>
   <223> oligonucleotide
<400> 79
   ccuccaacau caaggaagau ggcau 25
<210> 80
   <211> 25
   <212> RNA
   <213> artificial
<220>
   <223> oligonucleotide
<400> 80
   cuccaacauc aaggaagaug gcauu 25
<210> 81
   <211> 30
   <212> RNA
   <213> artificial
<220>
   <223> oligonucleotide
<400> 81
   cuccaacauc aaggaagaug gcauuucuag 30
<210> 82
   <211> 25
   <212> RNA
   <213> artificial
<220>
   <223> oligonucleotide
<400> 82
   uccaacauca aggaagaugg cauuu 25
<210> 83
   <211> 25
   <212> RNA
   <213> artificial
<220>
   <223> oligonucleotide
<400> 83
   ccaacaucaa ggaagauggc auuuc 25
<210> 84
   <211> 25
   <212> RNA
   <213> artificial
<220>
   <223> oligonucleotide
<400> 84
   caacaucaag gaagauggca uuucu 25
<210> 85
   <211> 25
   <212> RNA
   <213> artificial
<220>
   <223> oligonucleotide
<400> 85
   aacaucaagg aagauggcau uucua 25
<210> 86
   <211> 25
   <212> RNA
   <213> artificial
<220>
   <223> oligonucleotide
<400> 86
   acaucaagga agauggcauu ucuag 25
<210> 87
   <211> 30
   <212> RNA
   <213> artificial
<220>
   <223> oligonucleotide
<400> 87
   acaucaagga agauggcauu ucuaguuugg 30
<210> 88
   <211> 25
   <212> RNA
   <213> artificial
<220>
   <223> oligonucleotide
<400> 88
   acaucaagga agauggcauu ucuag 25
<210> 89
   <211> 25
   <212> RNA
   <213> artificial
<220>
   <223> oligonucleotide
<400> 89
   caucaaggaa gauggcauuu cuagu 25
<210> 90
   <211> 25
   <212> RNA
   <213> artificial
<220>
   <223> oligonucleotide
<400> 90
   aucaaggaag auggcauuuc uaguu 25
<210> 91
   <211> 25
   <212> RNA
   <213> artificial
<220>
   <223> oligonucleotide
<400> 91
   ucaaggaaga uggcauuucu aguuu 25
<210> 92
   <211> 20
   <212> RNA
   <213> artificial
<220>
   <223> oligonucleotide
<400> 92
   ucaaggaaga uggcauuucu 20
<210> 93
   <211> 25
   <212> RNA
   <213> artificial
<220>
   <223> oligonucleotide
<400> 93
   caaggaagau ggcauuucua guuug 25
<210> 94
   <211> 25
   <212> RNA
   <213> artificial
<220>
   <223> oligonucleotide
<400> 94
   aaggaagaug gcauuucuag uuugg 25
<210> 95
   <211> 25
   <212> RNA
   <213> artificial
<220>
   <223> oligonucleotide
<400> 95
   aggaagaugg cauuucuagu uugga 25
<210> 96
   <211> 25
   <212> RNA
   <213> artificial
<220>
   <223> oligonucleotide
<400> 96
   ggaagauggc auuucuaguu uggag 25
<210> 97
   <211> 25
   <212> RNA
   <213> artificial
<220>
   <223> oligonucleotide
<400> 97
   gaagauggca uuucuaguuu ggaga 25
<210> 98
   <211> 25
   <212> RNA
   <213> artificial
<220>
   <223> oligonucleotide
<400> 98
   aagauggcau uucuaguuug gagau 25
<210> 99
   <211> 25
   <212> RNA
   <213> artificial
<220>
   <223> oligonucleotide
<400> 99
   agauggcauu ucuaguuugg agaug 25
<210> 100
   <211> 25
   <212> RNA
   <213> artificial
<220>
   <223> oligonucleotide
<400> 100
   gauggcauuu cuaguuugga gaugg 25
<210> 101
   <211> 25
   <212> RNA
   <213> artificial
<220>
   <223> oligonucleotide
<400> 101
   auggcauuuc uaguuuggag auggc 25
<210> 102
   <211> 25
   <212> RNA
   <213> artificial
<220>
   <223> oligonucleotide
<400> 102
   uggcauuucu aguuuggaga uggca 25
<210> 103
   <211> 25
   <212> RNA
   <213> artificial
<220>
   <223> oligonucleotide
<400> 103
   ggcauuucua guuuggagau ggcag 25
<210> 104
   <211> 25
   <212> RNA
   <213> artificial
<220>
   <223> oligonucleotide
<400> 104
   gcauuucuag uuuggagaug gcagu 25
<210> 105
   <211> 25
   <212> RNA
   <213> artificial
<220>
   <223> oligonucleotide
<400> 105
   cauuucuagu uuggagaugg caguu 25
<210> 106
   <211> 25
   <212> RNA
   <213> artificial
<220>
   <223> oligonucleotide
<400> 106
   auuucuaguu uggagauggc aguuu 25
<210> 107
   <211> 25
   <212> RNA
   <213> artificial
<220>
   <223> oligonucleotide
<400> 107
   uuucuaguuu ggagauggca guuuc 25
<210> 108
   <211> 25
   <212> RNA
   <213> artificial
<220>
   <223> oligonucleotide
<400> 108
   uucuaguuug gagauggcag uuucc 25
<210> 109
   <211> 25
   <212> RNA
   <213> artificial
<220>
   <223> oligonucleotide
<400> 109
   ccauuguguu gaauccuuua acauu 25
<210> 110
   <211> 22
   <212> RNA
   <213> artificial
<220>
   <223> oligonucleotide
<400> 110
   ccauuguguu gaauccuuua ac 22
<210> 111
   <211> 20
   <212> RNA
   <213> artificial
<220>
   <223> oligonucleotide
<400> 111
   auuguguuga auccuuuaac 20
<210> 112
   <211> 20
   <212> RNA
   <213> artificial
<220>
   <223> oligonucleotide
<400> 112
   ccuguccuaa gaccugcuca 20
<210> 113
   <211> 25
   <212> RNA
   <213> artificial
<220>
   <223> oligonucleotide
<400> 113
   cuuuuggauu gcaucuacug uauag 25
<210> 114
   <211> 25
   <212> RNA
   <213> artificial
<220>
   <223> oligonucleotide
<400> 114
   cauucaacug uugccuccgg uucug 25
<210> 115
   <211> 24
   <212> RNA
   <213> artificial
<220>
   <223> oligonucleotide
<400> 115
   cuguugccuc cgguucugaa ggug 24
<210> 116
   <211> 31
   <212> RNA
   <213> artificial
<220>
   <223> oligonucleotide
<400> 116
   cauucaacug uugccuccgg uucugaaggu g 31
<210> 117
   <211> 25
   <212> RNA
   <213> artificial
<220>
   <223> oligonucleotide
<400> 117
   cugaaggugu ucuuguacuu caucc 25
<210> 118
   <211> 27
   <212> RNA
   <213> artificial
<220>
   <223> oligonucleotide
<400> 118
   uguauaggga cccuccuucc augacuc 27
<210> 119
   <211> 20
   <212> RNA
   <213> artificial
<220>
   <223> oligonucleotide
<400> 119
   aucccacuga uucugaauuc 20
<210> 120
   <211> 22
   <212> RNA
   <213> artificial
<220>
   <223> oligonucleotide
<400> 120
   uuggcucugg ccuguccuaa ga 22
<210> 121
   <211> 35
   <212> RNA
   <213> artificial
<220>
   <223> oligonucleotide
<400> 121
   aagaccugcu cagcuucuuc cuuagcuucc agcca 35
<210> 122
   <211> 23
   <212> RNA
   <213> artificial
<220>
   <223> oligonucleotide
<400> 122
   ugcauguucc agucguugug ugg 23
<210> 123
   <211> 25
   <212> RNA
   <213> artificial
<220>
   <223> oligonucleotide
<400> 123
   cacuauucca gucaaauagg ucugg 25
<210> 124
   <211> 25
   <212> RNA
   <213> artificial
<220>
   <223> oligonucleotide
<400> 124
   auuuaccaac cuucaggauc gagua 25
<210> 125
   <211> 21
   <212> RNA
   <213> artificial
<220>
   <223> oligonucleotide
<400> 125
   ggccuaaaac acauacacau a 21
<210> 126
   <211> 20
   <212> RNA
   <213> artificial
<220>
   <223> oligonucleotide
<400> 126
   ucagcuucug uuagccacug 20
<210> 127
   <211> 20
   <212> RNA
   <213> artificial
<220>
   <223> oligonucleotide
<400> 127
   uucagcuucu guuagccacu 20
<210> 128
   <211> 21
   <212> RNA
   <213> artificial
<220>
   <223> oligonucleotide
<400> 128
   uucagcuucu guuagccacu g 21
<210> 129
   <211> 21
   <212> RNA
   <213> artificial
<220>
   <223> oligonucleotide
<400> 129
   ucagcuucug uuagccacug a 21
<210> 130
   <211> 22
   <212> RNA
   <213> artificial
<220>
   <223> oligonucleotide
<400> 130
   uucagcuucu guuagccacu ga 22
<210> 131
   <211> 21
   <212> RNA
   <213> artificial
<220>
   <223> oligonucleotide
<400> 131
   ucagcuucug uuagccacug a 21
<210> 132
   <211> 22
   <212> RNA
   <213> artificial
<220>
   <223> oligonucleotide
<400> 132
   uucagcuucu guuagccacu ga 22
<210> 133
   <211> 22
   <212> RNA
   <213> artificial
<220>
   <223> oligonucleotide
<400> 133
   ucagcuucug uuagccacug au 22
<210> 134
   <211> 23
   <212> RNA
   <213> artificial
<220>
   <223> oligonucleotide
<400> 134
   uucagcuucu guuagccacu gau 23
<210> 135
   <211> 23
   <212> RNA
   <213> artificial
<220>
   <223> oligonucleotide
<400> 135
   ucagcuucug uuagccacug auu 23
<210> 136
   <211> 24
   <212> RNA
   <213> artificial
<220>
   <223> oligonucleotide
<400> 136
   uucagcuucu guuagccacu gauu 24
<210> 137
   <211> 24
   <212> RNA
   <213> artificial
<220>
   <223> oligonucleotide
<400> 137
   ucagcuucug uuagccacug auua 24
<210> 138
   <211> 24
   <212> RNA
   <213> artificial
<220>
   <223> oligonucleotide
<400> 138
   uucagcuucu guuagccacu gaua 24
<210> 139
   <211> 25
   <212> RNA
   <213> artificial
<220>
   <223> oligonucleotide
<400> 139
   ucagcuucug uuagccacug auuaa 25
<210> 140
   <211> 26
   <212> RNA
   <213> artificial
<220>
   <223> oligonucleotide
<400> 140
   uucagcuucu guuagccacu gauuaa 26
<210> 141
   <211> 26
   <212> RNA
   <213> artificial
<220>
   <223> oligonucleotide
<400> 141
   ucagcuucug uuagccacug auuaaa 26
<210> 142
   <211> 27
   <212> RNA
   <213> artificial
<220>
   <223> oligonucleotide
<400> 142
   uucagcuucu guuagccacu gauuaaa 27
<210> 143
   <211> 19
   <212> RNA
   <213> artificial
<220>
   <223> oligonucleotide
<400> 143
   cagcuucugu uagccacug 19
<210> 144
   <211> 21
   <212> RNA
   <213> artificial
<220>
   <223> oligonucleotide
<400> 144
   cagcuucugu uagccacuga u 21
<210> 145
   <211> 21
   <212> RNA
   <213> artificial
<220>
   <223> oligonucleotide
<400> 145
   agcuucuguu agccacugau u 21
<210> 146
   <211> 22
   <212> RNA
   <213> artificial
<220>
   <223> oligonucleotide
<400> 146
   cagcuucugu uagccacuga uu 22
<210> 147
   <211> 22
   <212> RNA
   <213> artificial
<220>
   <223> oligonucleotide
<400> 147
   agcuucuguu agccacugau ua 22
<210> 148
   <211> 23
   <212> RNA
   <213> artificial
<220>
   <223> oligonculeotide
<400> 148
   cagcuucugu uagccacuga uua 23
<210> 149
   <211> 23
   <212> RNA
   <213> artificial
<220>
   <223> oligonucleotide
<400> 149
   agcuucuguu agccacugau uaa 23
<210> 150
   <211> 24
   <212> RNA
   <213> artificial
<220>
   <223> oligonucleotide
<400> 150
   cagcuucugu uagccacuga uuaa 24
<210> 151
   <211> 24
   <212> RNA
   <213> artificial
<220>
   <223> oligonucleotide
<400> 151
   agcuucuguu agccacugau uaaa 24
<210> 152
   <211> 25
   <212> RNA
   <213> artificial
<220>
   <223> oligonucleotide
<400> 152
   cagcuucugu uagccacuga uuaaa 25
<210> 153
   <211> 24
   <212> RNA
   <213> artificial
<220>
   <223> oligonucleotide
<400> 153
   agcuucuguu agccacugau uaaa 24
<210> 154
   <211> 20
   <212> RNA
   <213> artificial
<220>
   <223> oligonucleotide
<400> 154
   agcuucuguu agccacugau 20
<210> 155
   <211> 20
   <212> RNA
   <213> artificial
<220>
   <223> oligonucleotide
<400> 155
   gcuucuguua gccacugauu 20
<210> 156
   <211> 21
   <212> RNA
   <213> artificial
<220>
   <223> oligonucleotide
<400> 156
   agcuucuguu agccacugau u 21
<210> 157
   <211> 21
   <212> RNA
   <213> artificial
<220>
   <223> oligonucleotide
<400> 157
   gcuucuguua gccacugauu a 21
<210> 158
   <211> 22
   <212> RNA
   <213> artificial
<220>
   <223> oligonculeotide
<400> 158
   agcuucuguu agccacugau ua 22
<210> 159
   <211> 22
   <212> RNA
   <213> artificial
<220>
   <223> oligonucleotide
<400> 159
   gcuucuguua gccacugauu aa 22
<210> 160
   <211> 23
   <212> RNA
   <213> artificial
<220>
   <223> oligonucleotide
<400> 160
   agcuucuguu agccacugau uaa 23
<210> 161
   <211> 23
   <212> RNA
   <213> artificial
<220>
   <223> oligonucleotide
<400> 161
   gcuucuguua gccacugauu aaa 23
<210> 162
   <211> 24
   <212> RNA
   <213> artificial
<220>
   <223> oligonucleotide
<400> 162
   agcuucuguu agccacugau uaaa 24
<210> 163
   <211> 23
   <212> RNA
   <213> artificial
<220>
   <223> oligonucleotide
<400> 163
   gcuucuguua gccacugauu aaa 23
<210> 164
   <211> 23
   <212> RNA
   <213> artificial
<220>
   <223> oligonucleotide
<400> 164
   ccauuuguau uuagcauguu ccc 23
<210> 165
   <211> 20
   <212> RNA
   <213> artificial
<220>
   <223> oligonucleotide
<400> 165
   agauaccauu uguauuuagc 20
<210> 166
   <211> 19
   <212> RNA
   <213> artificial
<220>
   <223> oligonucleotide
<400> 166
   gccauuucuc aacagaucu 19
<210> 167
   <211> 23
   <212> RNA
   <213> artificial
<220>
   <223> oligonucleotide
<400> 167
   gccauuucuc aacagaucug uca 23
<210> 168
   <211> 23
   <212> RNA
   <213> artificial
<220>
   <223> oligonucleotide
<400> 168
   auucucagga auuugugucu uuc 23
<210> 169
   <211> 21
   <212> RNA
   <213> artificial
<220>
   <223> oligonucleotide
<400> 169
   ucucaggaau uugugucuuu c 21
<210> 170
   <211> 18
   <212> RNA
   <213> artificial
<220>
   <223> oligonucleotide
<400> 170
   guucagcuuc uguuagcc 18
<210> 171
   <211> 21
   <212> RNA
   <213> artificial
<220>
   <223> oligonucleotide
<400> 171
   cugauuaaau aucuuuauau c 21
<210> 172
   <211> 18
   <212> RNA
   <213> artificial
<220>
   <223> oligonucleotide
<400> 172
   gccgccauuu cucaacag 18
<210> 173
   <211> 18
   <212> RNA
   <213> artificial
<220>
   <223> oligonucleotide
<400> 173
   guauuuagca uguuccca 18
<210> 174
   <211> 18
   <212> RNA
   <213> artificial
<220>
   <223> oligonucleotide
<400> 174
   caggaauuug ugucuuuc 18
<210> 175
   <211> 25
   <212> RNA
   <213> artificial
<220>
   <223> oligonucleotide
<400> 175
   gcuuuucuuu uaguugcugc ucuuu 25
<210> 176
   <211> 25
   <212> RNA
   <213> artificial
<220>
   <223> oligonucleotide
<400> 176
   cuuuucuuuu aguugcugcu cuuuu 25
<210> 177
   <211> 25
   <212> RNA
   <213> artificial
<220>
   <223> oligonucleotide
<400> 177
   uuuucuuuua guugcugcuc uuuuc 25
<210> 178
   <211> 25
   <212> RNA
   <213> artificial
<220>
   <223> oligonucleotide
<400> 178
   uuucuuuuag uugcugcucu uuucc 25
<210> 179
   <211> 25
   <212> RNA
   <213> artificial
<220>
   <223> oligonucleotide
<400> 179
   uucuuuuagu ugcugcucuu uucca 25
<210> 180
   <211> 25
   <212> RNA
   <213> artificial
<220>
   <223> oligonucleotide
<400> 180
   ucuuuuaguu gcugcucuuu uccag 25
<210> 181
   <211> 25
   <212> RNA
   <213> artificial
<220>
   <223> oligonucleotide
<400> 181
   cuuuuaguug cugcucuuuu ccagg 25
<210> 182
   <211> 25
   <212> RNA
   <213> artificial
<220>
   <223> oligonucleotide
<400> 182
   uuuuaguugc ugcucuuuuc caggu 25
<210> 183
   <211> 25
   <212> RNA
   <213> artificial
<220>
   <223> oligonucleotide
<400> 183
   uuuaguugcu gcucuuuucc agguu 25
<210> 184
   <211> 25
   <212> RNA
   <213> artificial
<220>
   <223> oligonucleotide
<400> 184
   uuaguugcug cucuuuucca gguuc 25
<210> 185
   <211> 25
   <212> RNA
   <213> artificial
<220>
   <223> oligonucleotide
<400> 185
   uaguugcugc ucuuuuccag guuca 25
<210> 186
   <211> 25
   <212> RNA
   <213> artificial
<220>
   <223> oligonucleotide
<400> 186
   aguugcugcu cuuuuccagg uucaa 25
<210> 187
   <211> 25
   <212> RNA
   <213> artificial
<220>
   <223> oligonucleotide
<400> 187
   guugcugcuc uuuuccaggu ucaag 25
<210> 188
   <211> 25
   <212> RNA
   <213> artificial
<220>
   <223> oligonucleotide
<400> 188
   uugcugcucu uuuccagguu caagu 25
<210> 189
   <211> 25
   <212> RNA
   <213> artificial
<220>
   <223> oligonucleotide
<400> 189
   ugcugcucuu uuccagguuc aagug 25
<210> 190
   <211> 25
   <212> RNA
   <213> artificial
<220>
   <223> oligonucleotide
<400> 190
   gcugcucuuu uccagguuca agugg 25
<210> 191
   <211> 25
   <212> RNA
   <213> artificial
<220>
   <223> oligonucleotide
<400> 191
   cugcucuuuu ccagguucaa guggg 25
<210> 192
   <211> 25
   <212> RNA
   <213> artificial
<220>
   <223> oligonucleotide
<400> 192
   ugcucuuuuc cagguucaag uggga 25
<210> 193
   <211> 25
   <212> RNA
   <213> artificial
<220>
   <223> oligonucleotide
<400> 193
   gcucuuuucc agguucaagu gggac 25
<210> 194
   <211> 25
   <212> RNA
   <213> artificial
<220>
   <223> oligonucleotide
<400> 194
   cucuuuucca gguucaagug ggaua 25
<210> 195
   <211> 25
   <212> RNA
   <213> artificial
<220>
   <223> oligonucleotide
<400> 195
   ucuuuuccag guucaagugg gauac 25
<210> 196
   <211> 25
   <212> RNA
   <213> artificial
<220>
   <223> oligonucleotide
<400> 196
   cuuuuccagg uucaaguggg auacu 25
<210> 197
   <211> 25
   <212> RNA
   <213> artificial
<220>
   <223> oligonucleotide
<400> 197
   uuuuccaggu ucaaguggga uacua 25
<210> 198
   <211> 25
   <212> RNA
   <213> artificial
<220>
   <223> oligonucleotide
<400> 198
   uuuccagguu caagugggau acuag 25
<210> 199
   <211> 25
   <212> RNA
   <213> artificial
<220>
   <223> oligonucleotide
<400> 199
   uuccagguuc aagugggaua cuagc 25
<210> 200
   <211> 25
   <212> RNA
   <213> artificial
<220>
   <223> oligonucleotide
<400> 200
   uccagguuca agugggauac uagca 25
<210> 201
   <211> 25
   <212> RNA
   <213> artificial
<220>
   <223> oligonucleotide
<400> 201
   ccagguucaa gugggauacu agcaa 25
<210> 202
   <211> 25
   <212> RNA
   <213> artificial
<220>
   <223> oligonucleotide
<400> 202
   cagguucaag ugggauacua gcaau 25
<210> 203
   <211> 25
   <212> RNA
   <213> artificial
<220>
   <223> oligonucleotide
<400> 203
   agguucaagu gggauacuag caaug 25
<210> 204
   <211> 25
   <212> RNA
   <213> artificial
<220>
   <223> oligonucleotide
<400> 204
   gguucaagug ggauacuagc aaugu 25
<210> 205
   <211> 25
   <212> RNA
   <213> artificial
<220>
   <223> oligonucleotide
<400> 205
   guucaagugg gauacuagca auguu 25
<210> 206
   <211> 25
   <212> RNA
   <213> artificial
<220>
   <223> oligonucleotide
<400> 206
   uucaaguggg auacuagcaa uguua 25
<210> 207
   <211> 25
   <212> RNA
   <213> artificial
<220>
   <223> oligonucleotide
<400> 207
   ucaaguggga uacuagcaau guuau 25
<210> 208
   <211> 25
   <212> RNA
   <213> artificial
<220>
   <223> oligonucleotide
<400> 208
   caagugggau acuagcaaug uuauc 25
<210> 209
   <211> 25
   <212> RNA
   <213> artificial
<220>
   <223> oligonucleotide
<400> 209
   aagugggaua cuagcaaugu uaucu 25
<210> 210
   <211> 25
   <212> RNA
   <213> artificial
<220>
   <223> oligonucleotide
<400> 210
   agugggauac uagcaauguu aucug 25
<210> 211
   <211> 25
   <212> RNA
   <213> artificial
<220>
   <223> oligonucleotide
<400> 211
   gugggauacu agcaauguua ucugc 25
<210> 212
   <211> 25
   <212> RNA
   <213> artificial
<220>
   <223> oligonucleotide
<400> 212
   ugggauacua gcaauguuau cugcu 25
<210> 213
   <211> 25
   <212> RNA
   <213> artificial
<220>
   <223> oligonucleotide
<400> 213
   gggauacuag caauguuauc ugcuu 25
<210> 214
   <211> 25
   <212> RNA
   <213> artificial
<220>
   <223> oligonucleotide
<400> 214
   ggauacuagc aauguuaucu gcuuc 25
<210> 215
   <211> 25
   <212> RNA
   <213> artificial
<220>
   <223> oligonucleotide
<400> 215
   gauacuagca auguuaucug cuucc 25
<210> 216
   <211> 25
   <212> RNA
   <213> artificial
<220>
   <223> oligonucleotide
<400> 216
   auacuagcaa uguuaucugc uuccu 25
<210> 217
   <211> 25
   <212> RNA
   <213> artificial
<220>
   <223> oligonucleotide
<400> 217
   uacuagcaau guuaucugcu uccuc 25
<210> 218
   <211> 25
   <212> RNA
   <213> artificial
<220>
   <223> oligonucleotide
<400> 218
   acuagcaaug uuaucugcuu ccucc 25
<210> 219
   <211> 25
   <212> RNA
   <213> artificial
<220>
   <223> oligonucleotide
<400> 219
   cuagcaaugu uaucugcuuc cucca 25
<210> 220
   <211> 25
   <212> RNA
   <213> artificial
<220>
   <223> oligonucleotide
<400> 220
   uagcaauguu aucugcuucc uccaa 25
<210> 221
   <211> 25
   <212> RNA
   <213> artificial
<220>
   <223> oligonucleotide
<400> 221
   agcaauguua ucugcuuccu ccaac 25
<210> 222
   <211> 25
   <212> RNA
   <213> artificial
<220>
   <223> oligonucleotide
<400> 222
   gcaauguuau cugcuuccuc caacc 25
<210> 223
   <211> 25
   <212> RNA
   <213> artificial
<220>
   <223> oligonucleotide
<400> 223
   caauguuauc ugcuuccucc aacca 25
<210> 224
   <211> 25
   <212> RNA
   <213> artificial
<220>
   <223> oligonucleotide
<400> 224
   aauguuaucu gcuuccucca accau 25
<210> 225
   <211> 25
   <212> RNA
   <213> artificial
<220>
   <223> oligonucleotide
<400> 225
   auguuaucug cuuccuccaa ccaua 25
<210> 226
   <211> 25
   <212> RNA
   <213> artificial
<220>
   <223> oligonucleotide
<400> 226
   uguuaucugc uuccuccaac cauaa 25
<210> 227
   <211> 25
   <212> RNA
   <213> artificial
<220>
   <223> oligonucleotide
<400> 227
   guuaucugcu uccuccaacc auaaa 25
<210> 228
   <211> 19
   <212> RNA
   <213> artificial
<220>
   <223> oligonucleotide
<400> 228
   gcugcucuuu uccagguuc 19
<210> 229
   <211> 20
   <212> RNA
   <213> artificial
<220>
   <223> oligonucleotide
<400> 229
   ucuuuuccag guucaagugg 20
<210> 230
   <211> 19
   <212> RNA
   <213> artificial
<220>
   <223> oligonucleotide
<400> 230
   agguucaagu gggauacua 19
<210> 231
   <211> 21
   <212> RNA
   <213> artificial
<220>
   <223> oligonucleotide
<400> 231
   caauuuuucc cacucaguau u 21
<210> 232
   <211> 19
   <212> RNA
   <213> artificial
<220>
   <223> oligonucleotide
<400> 232
   uugaaguucc uggagucuu 19
<210> 233
   <211> 22
   <212> RNA
   <213> artificial
<220>
   <223> oligonucleotide
<400> 233
   uccucaggag gcagcucuaa au 22
<210> 234
   <211> 26
   <212> RNA
   <213> artificial
<220>
   <223> oligonucleotide
<400> 234
   gcgcugguca caaaauccug uugaac 26
<210> 235
   <211> 27
   <212> RNA
   <213> artificial
<220>
   <223> oligonucleotide
<400> 235
   cacuugcuug aaaaggucua caaagga 27
<210> 236
   <211> 26
   <212> RNA
   <213> artificial
<220>
   <223> oligonucleotide
<400> 236
   ggugaauaac uuacaaauuu ggaagc 26

## Description

### Field

The invention relates to the field of genetics, more specifically human genetics. The invention in particular relates to the modulation of splicing of the human Duchenne Muscular Dystrophy pre-mRNA.

### Background of the invention

Myopathies are disorders that result in functional impairment of muscles. Muscular dystrophy (MD) refers to genetic diseases that are characterized by progressive weakness and degeneration of skeletal muscles. Duchenne muscular dystrophy (DMD) and Becker muscular dystrophy (BMD) are the most common childhood forms of muscular dystrophy. They are recessive disorders and because the gene responsible for DMD and BMD resides on the X-chromosome, mutations mainly affect males with an incidence of about 1 in 3500 boys.

DMD and BMD are caused by genetic defects in the DMD gene encoding dystrophin, a muscle protein that is required for interactions between the cytoskeleton and the extracellular matrix to maintain muscle fiber stability during contraction. DMD is a severe, lethal neuromuscular disorder resulting in a dependency on wheelchair support before the age of 12 and DMD patients often die before the age of thirty due to respiratory- or heart failure. In contrast, BMD patients often remain ambulatory until later in life, and have near normal life expectancies. DMD mutations in the DMD gene are mainly characterized by frame shifting insertions or deletions or nonsense point mutations, resulting in the absence of functional dystrophin. BMD mutations in general keep the reading frame intact, allowing synthesis of a partly functional dystrophin.

During the last decade, specific modification of splicing in order to restore the disrupted reading frame of the DMD transcript has emerged as a promising therapy for Duchenne muscular dystrophy (DMD) (van Ommen, van Deutekom, Aartsma-Rus, Curr Opin Mol Ther. 2008;10(2):140-9, Yokota, Duddy, Partidge, Acta Myol. 2007;26(3): 179-84, van Deutekom et al., N Engl J Med. 2007;357(26):2677-86).

Using antisense oligonucleotides (AONs) interfering with splicing signals the skipping of specific exons can be induced in the DMD pre-mRNA, thus restoring the open reading frame and converting the severe DMD into a milder BMD phenotype (van Deutekom et al. Hum Mol Genet. 2001; 10: 1547-54; Aartsma-Rus et al., Hum Mol Genet 2003; 12(8):907-14.). Exon skipping by interfering with the exonic splicer enhancer (ESE) sites of the DMD gene was demonstrated in WO 2007/135105 A1. A splicing enhancer sequence for exon 45 of the DMD gene was identified in EP1191098, targeting of this site seem to result in exon 45 skipping in a mini gene construct. More recently, several antisense oligonucleotides targeting exon 45 were disclosed in WO 2010/048586. *In vivo* proof-of-concept was first obtained in the *mdx* mouse model, which is dystrophin-deficient due to a nonsense mutation in exon 23. Intramuscular and intravenous injections of AONs targeting the mutated exon 23 restored dystrophin expression for at least three months (Lu et al. Nat Med. 2003; 8: 1009-14; Lu et al., Proc Natl Acad Sci USA. 2005;102(1):198-203). This was accompanied by restoration of dystrophin-associated proteins at the fiber membrane as well as functional improvement of the treated muscle. *In vivo* skipping of human exons has also been achieved in the hDMD mouse model, which contains a complete copy of the human DMD gene integrated in chromosome 5 of the mouse (Bremmer-Bout et al. Molecular Therapy. 2004; 10: 232-40; 't Hoen et al. J Biol Chem. 2008; 283: 5899-907).

As the majority of DMD patients have deletions that cluster in hotspot regions, the skipping of a small number of exons is applicable to relatively large numbers of patients. The actual applicability of exon skipping can be determined for deletions, duplications and point mutations reported in DMD mutation databases such as the Leiden DMD mutation database available at www.dmd.nl. Therapeutic skipping of exon 45 of the DMD pre-mRNA would restore the open reading frame of DMD patients having deletions including but not limited to exons 12-44, 18-44, 44, 46, 46-47, 46-48, 46-49, 46-51, 46-53, 46-55, 46-59, 46-60 of the DMD pre-mRNA, occurring in a total of 16 % of all DMD patients with a deletion (Aartsma-Rus and van Deutekom, 2007, Antisense Elements (Genetics) Research Focus, 2007 Nova Science Publishers, Inc). Furthermore, for some DMD patients the simultaneous skipping of one of more exons in addition to exon 45, such as exons 51 or 53 is required to restore the correct reading frame. None-limiting examples include patients with a deletion of exons 46-50 requiring the co-skipping of exons 45 and 51, or with a deletion of exons 46-52 requiring the co-skipping of exons 45 and 53.

Recently, a first-in-man study was successfully completed where an AON inducing the skipping of exon 51 was injected into a small area of the tibialis anterior muscle of four DMD patients. Novel dystrophin expression was observed in the majority of muscle fibers in all four patients treated, and the AON was safe and well tolerated (van Deutekom et al. N Engl J Med. 2007; 357: 2677-86).

Most AONs studied contain up to 20 nucleotides, and it has been argued that this relatively short size improves the tissue distribution and/or cell penetration of an AON. However, such short AONs will result in a limited specificity due to an increased risk for the presence of identical sequences elsewhere in the genome, and a limited target binding or target affinity due to a low free energy of the AON-target complex. Therefore the inventors decided to design new and optionally improved oligonucleotides that would not exhibit all of these drawbacks.

### Description of the invention

### Method

In a first aspect, the invention provides a method for inducing and/or promoting skipping of exon 45 of DMD pre-mRNA in a patient, preferably in an isolated cell of said patient, the method comprising providing said cell and/or said patient with a molecule that binds to a continuous stretch of at least 21 nucleotides within said exon wherein said stretch and said molecule are as defined in claim 1.

Accordingly, a method is herewith provided for inducing and/or promoting skipping of exon 45 of DMD pre-mRNA, preferably in an isolated cell of a patient, the method comprising providing said cell and/or said patient with a molecule that binds to a continuous stretch of at least 21 nucleotides within said exon wherein said stretch and said molecule are as defined in claim 1.

It is to be understood that said method encompasses an *in vitro*, *in vivo* or *ex vivo* method.

As defined herein a DMD pre-mRNA preferably means the pre-mRNA of a DMD gene of a DMD or BMD patient. The DMD gene or protein corresponds to the dystrophin gene or protein.

A patient is preferably intended to mean a patient having DMD or BMD as later defined herein or a patient susceptible to develop DMD or BMD due to his or her genetic background.

Exon skipping refers to the induction in a cell of a mature mRNA that does not contain a particular exon that is normally present therein. Exon skipping is achieved by providing a cell expressing the pre-mRNA of said mRNA with a molecule capable of interfering with sequences such as, for example, the splice donor or splice acceptor sequence that are both required for allowing the enzymatic process of splicing, or a molecule that is capable of interfering with an exon inclusion signal required for recognition of a stretch of nucleotides as an exon to be included in the mRNA. The term pre-mRNA refers to a non-processed or partly processed precursor mRNA that is synthesized from a DNA template in the cell nucleus by transcription.

Within the context of the invention inducing and/or promoting skipping of an exon as indicated herein means that at least 1%, 10%, 20%, 30%, 40%,50%, 60%, 70%, 80%, 90% or more of the DMD mRNA in one or more (muscle) cells of a treated patient will not contain said exon. This is preferably assessed by PCR as described in the examples.

Preferably, an in vitro method of the invention by inducing or promoting skipping of exon 45 of the DMD pre-mRNA in one or more cells of a patient provides said patient with a functional dystrophin protein and/or decreases the production of an aberrant dystrophin protein in said patient. Therefore a preferred method is a method, wherein a cell of said patient is provided with a functional dystrophin protein and/or wherein the production of an aberrant dystrophin protein in a cell of said patient is decreased

Decreasing the production of an aberrant dystrophin may be assessed at the mRNA level and preferably means that 99%, 90%, 80%, 70%, 60%, 50%, 40%, 30%, 20%, 10%, 5% or less of the initial amount of aberrant dystrophin mRNA, is still detectable by RT PCR. An aberrant dystrophin mRNA or protein is also referred to herein as a non-functional dystrophin mRNA or protein. A non functional dystrophin protein is preferably a dystrophin protein which is not able to bind actin and/or members of the DGC protein complex. A non-functional dystrophin protein or dystrophin mRNA does typically not have, or does not encode a dystrophin protein with an intact C-terminus of the protein.

Increasing the production of a functional dystrophin in a cell of said patient may be assessed at the mRNA level (by RT-PCR analysis) and preferably means that a detectable amount of a functional dystrophin mRNA is detectable by RT PCR. In another embodiment, 1%, 5%, 10%, 20%, 30%, 40%, 50%, 60%, 70%, 80%, 90% or more of the detectable dystrophin mRNA is a functional dystrophin mRNA. Increasing the production of a functional dystrophin in a cell of said patient may be assessed at the protein level (by immunofluorescence and western blot analyses) and preferably means that a detectable amount of a functional dystrophin protein is detectable by immunofluorescence or western blot analysis. In another embodiment, 1%, 5%, 10%, 20%, 30%, 40%, 50%, 60%, 70%, 80%, 90% or more of the detectable dystrophin protein is a functional dystrophin protein.

As defined herein, a functional dystrophin is preferably a wild type dystrophin corresponding to a protein having the amino acid sequence as identified in SEQ ID NO: 1. A functional dystrophin is preferably a dystrophin, which has an actin binding domain in its N terminal part (first 240 amino acids at the N terminus), a cystein-rich domain (amino acid 3361 till 3685) and a C terminal domain (last 325 amino acids at the C terminus) each of these domains being present in a wild type dystrophin as known to the skilled person. The amino acids indicated herein correspond to amino acids of the wild type dystrophin being represented by SEQ ID NO:1. In other words, a functional dystrophin is a dystrophin which exhibits at least to some extent an activity of a wild type dystrophin. "At least to some extent" preferably means at least 10%, 20%, 30%, 40%, 50%, 60%, 70%, 80%, 90%, 95% or 100% of a corresponding activity of a wild type functional dystrophin. In this context, an activity of a functional dystrophin is preferably binding to actin and to the dystrophin-associated glycoprotein complex (DGC) (Aartsma-Rus A et al, (2006), Entries in the leiden Duchenne Muscular Dystrophy mutation database: an overview of mutation types and paradoxical cases that confirm the reading-frame rule, Muscle Nerve, 34: 135-144). Binding of dystrophin to actin and to the DGC complex may be visualized by either co-immunoprecipitation using total protein extracts or immunofluorescence analysis of cross-sections, from a muscle biopsy, as known to the skilled person.

Individuals or patients suffering from Duchenne muscular dystrophy typically have a mutation in the DMD gene that prevent synthesis of the complete dystrophin protein, i.e of a premature stop prevents the synthesis of the C-terminus. In Becker muscular dystrophy the DMD gene also comprises a mutation compared to the wild type gene but the mutation does typically not induce a premature stop and the C-terminus is typically synthesized. As a result a functional dystrophin protein is synthesized that has at least the same activity in kind as the wild type protein, not although not necessarily the same amount of activity. The genome of a BMD individual typically encodes a dystrophin protein comprising the N terminal part (first 240 amino acids at the N terminus), a cystein-rich domain (amino acid 3361 till 3685) and a C terminal domain (last 325 amino acids at the C terminus) but its central rod shaped domain may be shorter than the one of a wild type dystrophin (Aartsma-Rus A et al, (2006), Entries in the leiden Duchenne Muscular Dystrophy mutation database: an overview of mutation types and paradoxical cases that confirm the reading-frame rule, Muscle Nerve, 34: 135-144). Exon -skipping for the treatment of DMD is typically directed to overcome a premature stop in the pre-mRNA by skipping an exon in the rod-shaped domain to correct the reading frame and allow synthesis of the remainder of the dystrophin protein including the C-terminus, albeit that the protein is somewhat smaller as a result of a smaller rod domain. In a preferred embodiment, an individual having DMD and being treated by a method as defined herein will be provided a dystrophin which exhibits at least to some extent an activity of a wild type dystrophin. More preferably, if said individual is a Duchenne patient or is suspected to be a Duchenne patient, a functional dystrophin is a dystrophin of an individual having BMD: typically said dystrophin is able to interact with both actin and the DGC, but its central rod shaped domain may be shorter than the one of a wild type dystrophin (Aartsma-Rus A et al, (2006), Entries in the leiden Duchenne Muscular Dystrophy mutation database: an overview of mutation types and paradoxical cases that confirm the reading-frame rule, Muscle Nerve, 34: 135-144). The central rod-shaped domain of wild type dystrophin comprises 24 spectrin-like repeats (Aartsma-Rus A et al, (2006), Entries in the leiden Duchenne Muscular Dystrophy mutation database: an overview of mutation types and paradoxical cases that confirm the reading-frame rule, Muscle Nerve, 34: 135-144). For example, a central rod-shaped domain of a dystrophin as provided herein may comprise 5 to 23, 10 to 22 or 12 to 18 spectrin-like repeats as long as it can bind to actin and to DGC.

An in vitro method of the invention may alleviate one or more characteristics of a muscle cell from a DMD patient comprising deletions including but not limited to exons 12-44, 18-44, 44, 46, 46-47, 46-48, 46-49, 46-51, 46-53, 46-55, 46-59, 46-60 of the DMD pre-mRNA of said patient (Aartsma-Rus and van Deutekom, 2007, Antisense Elements (Genetics) Research Focus, 2007 Nova Science Publishers, Inc) as well as from DMD patients requiring the simultaneous skipping of one of more exons in addition to exon 45 including but not limited to patients with a deletion of exons 46-50 requiring the co-skipping of exons 45 and 51, or with a deletion of exons 46-52 requiring the co-skipping of exons 45 and 53.

In a preferred method, one or more symptom(s) or characteristic(s) of a myogenic cell or muscle cell from a DMD patient is/are alleviated. Such symptoms or characteristics may be assessed at the cellular, tissue level or on the patient self.

An alleviation of one or more symptoms or characteristics may be assessed by any of the following assays on a myogenic cell or muscle cell from a patient: reduced calcium uptake by muscle cells, decreased collagen synthesis, altered morphology, altered lipid biosynthesis, decreased oxidative stress, and/or improved muscle fiber function, integrity, and/or survival. These parameters are usually assessed using immunofluorescence and/or histochemical analyses of cross sections of muscle biopsies.

The improvement of muscle fiber function, integrity and/or survival may also be assessed using at least one of the following assays: a detectable decrease of creatine kinase in blood, a detectable decrease of necrosis of muscle fibers in a biopsy cross-section of a muscle suspected to be dystrophic, and/or a detectable increase of the homogeneity of the diameter of muscle fibers in a biopsy cross-section of a muscle suspected to be dystrophic. Each of these assays is known to the skilled person.

Creatine kinase may be detected in blood as described in Hodgetts et al (Hodgetts S., et al, (2006), Neuromuscular Disorders, 16: 591-602.2006). A detectable decrease in creatine kinase may mean a decrease of 5%, 10%, 20%, 30%, 40%, 50%, 60%, 70%, 80%, 90% or more compared to the concentration of creatine kinase in a same DMD patient before treatment.

A detectable decrease of necrosis of muscle fibers is preferably assessed in a muscle biopsy, more preferably as described in Hodgetts et al (Hodgetts S., et al, (2006), Neuromuscular Disorders, 16: 591-602.2006) using biopsy cross-sections. A detectable decrease of necrosis may be a decrease of 5%, 10%, 20%, 30%, 40%, 50%, 60%, 70%, 80%, 90% or more of the area wherein necrosis has been identified using biopsy cross-sections. The decrease is measured by comparison to the necrosis as assessed in a same DMD patient before treatment.

A detectable increase of the homogeneity of the diameter of muscle fibers is preferably assessed in a muscle biopsy cross-section, more preferably as described in Hodgetts et al (Hodgetts S., et al, (2006), Neuromuscular Disorders, 16: 591-602.2006). The increase is measured by comparison to the homogeneity of the diameter of muscle fibers in a muscle biopsy cross-section of a same DMD patient before treatment.

An alleviation of one or more symptoms or characteristics may be assessed by any of the following assays on the patient self: prolongation of time to loss of walking, improvement of muscle strength, improvement of the ability to lift weight, improvement of the time taken to rise from the floor, improvement in the nine-meter walking time, improvement in the time taken for four-stairs climbing, improvement of the leg function grade, improvement of the pulmonary function, improvement of cardiac function, improvement of the quality of life. Each of these assays is known to the skilled person. As an example, the publication of Manzur at al (Manzur AY et al, (2008), Glucocorticoid corticosteroids for Duchenne muscular dystrophy (review), Wiley publishers, The Cochrane collaboration.) gives an extensive explanation of each of these assays. For each of these assays, as soon as a detectable improvement or prolongation of a parameter measured in an assay has been found, it will preferably mean that one or more symptoms of Duchenne Muscular Dystrophy has been alleviated in an individual using a method of the invention. Detectable improvement or prolongation is preferably a statistically significant improvement or prolongation as described in Hodgetts et al (Hodgetts S., et al, (2006), Neuromuscular Disorders, 16: 591-602.2006). Alternatively, the alleviation of one or more symptom(s) of Duchenne Muscular Dystrophy may be assessed by measuring an improvement of a muscle fiber function, integrity and/or survival as later defined herein.

A treatment may have a duration of at least one week, at least one month, at least several months, at least one year, at least 2, 3, 4, 5, 6 years or more. The frequency of administration of an oligonucleotide, composition, compound of the invention may depend on several parameters such as the age of the patient, the type of mutation, the number of molecules (dose), the formulation of said molecule. The frequency may be ranged between at least once in a two weeks, or three weeks or four weeks or five weeks or a longer time period. Each molecule or oligonucleotide or equivalent thereof as defined herein for use according to the invention may be suitable for direct administration to a cell, tissue and/or an organ *in vivo* of individuals affected by or at risk of developing DMD and may be administered directly *in vivo*, *ex vivo* or *in vitro*. An oligonucleotide as used herein may be suitable for administration to a cell, tissue and/or an organ *in vivo* of individuals affected by or at risk of developing DMD, and may be administered *in vivo*, *ex vivo* or *in vitro*. Said oligonucleotide may be directly or indirectly administrated to a cell, tissue and/or an organ *in vivo* of an individual affected by or at risk of developing DMD, and may be administered directly or indirectly *in vivo*, *ex vivo* or *in vitro*. As Duchenne muscular dystrophy has a pronounced phenotype in muscle cells, it is preferred that said cells are muscle cells, it is further preferred that said tissue is a muscular tissue and/or it is further preferred that said organ comprises or consists of a muscular tissue. A preferred organ is the heart. Preferably said cells comprise a gene encoding a mutant dystrophin protein. Preferably said cells are cells of an individual suffering from DMD.

A molecule or oligonucleotide or equivalent thereof can be delivered as is to a cell. When administering said molecule, oligonucleotide or equivalent thereof to an individual, it is preferred that it is dissolved in a solution that is compatible with the delivery method. For intravenous, subcutaneous, intramuscular, intrathecal and/or intraventricular administration it is preferred that the solution is a physiological salt solution. Particularly preferred for a method of the invention is the use of an excipient that will further enhance delivery of said molecule, oligonucleotide or functional equivalent thereof as defined herein, to a cell and into a cell, preferably a muscle cell. Preferred excipient are defined in the section entitled "pharmaceutical composition". In vitro, we obtained very good results using polyethylenimine (PEI, ExGen500, MBI Fermentas) as shown in the example.

In a preferred method of the invention, an additional molecule is used which is able to induce and/or promote skipping of a distinct exon of the DMD pre-mRNA of a patient. Preferably, the second exon is selected from: exon 7, 44, 46, 51, 53, 59, 67 of the dystrophin pre-mRNA of a patient. Molecules which can be used are depicted in table 2. Preferred molecules comprise or consist of any of the oligonucleotides as disclosed in table 2. Several oligonucleotides may also be used in combination. This way, inclusion of two or more exons of a DMD pre-mRNA in mRNA produced from this pre-mRNA is prevented. This embodiment is further referred to as double- or multi-exon skipping (Aartsma-Rus A, Janson AA, Kaman WE, et al. Antisense-induced multiexon skipping for Duchenne muscular dystrophy makes more sense. Am J Hum Genet 2004;74(1):83-92, Aartsma-Rus A, Kaman WE, Weij R, den Dunnen JT, van Ommen GJ, van Deutekom JC. Exploring the frontiers of therapeutic exon skipping for Duchenne muscular dystrophy by double targeting within one or multiple exons. Mol Ther 2006;14(3):401-7). In most cases double-exon skipping results in the exclusion of only the two targeted exons from the dystrophin pre-mRNA. However, in other cases it was found that the targeted exons and the entire region in between said exons in said pre-mRNA were not present in the produced mRNA even when other exons (intervening exons) were present in such region. This multi-skipping was notably so for the combination of oligonucleotides derived from the DMD gene, wherein one oligonucleotide for exon 45 and one oligonucleotide for exon 51 was added to a cell transcribing the DMD gene. Such a set-up resulted in mRNA being produced that did not contain exons 45 to 51. Apparently, the structure of the pre-mRNA in the presence of the mentioned oligonucleotides was such that the splicing machinery was stimulated to connect exons 44 and 52 to each other.

It is possible to specifically promote the skipping of also the intervening exons by providing a linkage between the two complementary oligonucleotides. Hence, in one embodiment stretches of nucleotides complementary to at least two dystrophin exons are separated by a linking moiety. The at least two stretches of nucleotides are thus linked in this embodiment so as to form a single molecule.

In case, more than one compounds are used in a method of the invention, said compounds can be administered to an individual in any order. In one embodiment, said compounds are administered simultaneously (meaning that said compounds are administered within 10 hours, preferably within one hour). This is however not necessary. In another embodiment, said compounds are administered sequentially.

### Molecule

In a second aspect, there is provided a molecule for use in a method as described in the previous section entitled "Method". This molecule preferably comprises or consists of an oligonucleotide, Said oligonucleotide is preferably an antisense oligonucleotide (AON) or antisense oligoribonucleotide.

It was found by the present investigators that especially exon 45 is specifically skipped at a high frequency using a molecule that binds to a continuous stretch of at least 21 nucleotides within said exon as defined in claim 1. Although this effect can be associated with a higher binding affinity of said molecule, compared to a molecule that binds to a continuous stretch of less than 21 nucleotides, there could be other intracellular parameters involved that favor thermodynamic, kinetic, or structural characteristics of the hybrid duplex. In a preferred embodiment, a molecule that binds to a continuous stretch of at least 21, 25, 30, 35, 40, 45, 50 nucleotides within said exon is used.

In a preferred embodiment, a molecule or an oligonucleotide of the invention which comprises a sequence that is complementary to a part of exon 45 of DMD pre-mRNA is such that the complementary part is at least 50% of the length of the oligonucleotide of the invention, more preferably at least 60%, even more preferably at least 70%, even more preferably at least 80%, even more preferably at least 90% or even more preferably at least 95%, or even more preferably 98% and most preferably up to 100%. "A part of exon 45" preferably means a stretch of at least 21 nucleotides. In a most preferred embodiment, an oligonucleotide of the invention consists of a sequence that is complementary to part of exon 45 dystrophin pre-mRNA as defined herein. Alternatively, an oligonucleotide may comprise a sequence that is complementary to part of exon 45 dystrophin pre-mRNA as defined herein and additional flanking sequences. In a more preferred embodiment, the length of said complementary part of said oligonucleotide is of at least 21, 22, 23, 24, 25, 26, 27, 28 , 29, 30, 31, 32, 33, 34, 35, 36, 37, 38, 39, 40, 41, 42, 43, 44, 45, 46, 47, 48, 49, 50 nucleotides. Several types of flanking sequences may be used. Preferably, additional flanking sequences are used to modify the binding of a protein to said molecule or oligonucleotide, or to modify a thermodynamic property of the oligonucleotide, more preferably to modify target RNA binding affinity. In another preferred embodiment, additional flanking sequences are complementary to sequences of the DMD pre-mRNA which are not present in exon 45. Such flanking sequences are preferably complementary to sequences comprising or consisting of the splice site acceptor or donor consensus sequences of exon 45. In a preferred embodiment, such flanking sequences are complementary to sequences comprising or consisting of sequences of an intron of the DMD pre-mRNA which is adjacent to exon 45; i.e. intron 44 or 45.

A continuous stretch of at least 21, 25, 30, 35, 40, 45, 50 nucleotides within exon 45 is selected from the sequence:

It was found that a molecule that binds to a nucleotide sequence comprising or consisting of a continuous stretch of at least 21, 25, 30, 35, 40, 45, 50 nucleotides of SEQ ID NO. 2 results in highly efficient skipping of exon 45 in a cell provided with this molecule. Molecules that bind to a nucleotide sequence comprising a continuous stretch of less than 21 nucleotides of SEQ ID NO:2 were found to induce exon skipping in a less efficient way than the molecules of the invention. Therefore, in a preferred embodiment, a method is provided wherein a molecule binds to a continuous stretch of at least 21, 25, 30, 35 nucleotides within SEQ ID NO:2. Contrary to what was generally thought, the inventors surprisingly found that a higher specificity and efficiency of exon skipping may be reached using an oligonucleotides having a length of at least 21 nucleotides. None of the indicated sequences is derived from conserved parts of splice-junction sites. Therefore, said molecule is not likely to mediate differential splicing of other exons from the DMD pre-mRNA or exons from other genes.

In one embodiment, a molecule of the invention capable of interfering with the inclusion of exon 45 of the DMD pre-mRNA is a compound molecule that binds to the specified sequence, or a protein such as an RNA-binding protein or a non-natural zinc-finger protein that has been modified to be able to bind to the indicated nucleotide sequence on a RNA molecule. Methods for screening compound molecules that bind specific nucleotide sequences are for example disclosed in PCT/NL01/00697 and US patent 6875736, which are herein enclosed by reference. Methods for designing RNA-binding Zinc-finger proteins that bind specific nucleotide sequences are disclosed by Friesen and Darby, Nature Structural Biology 5: 543- 546 (1998) which is herein enclosed by reference.

In a further embodiment, a molecule of the invention capable of interfering with the inclusion of exon 45 of the DMD pre-mRNA comprises an antisense oligonucleotide that is complementary to and can base-pair with the coding strand of the pre-mRNA of the DMD gene. Said antisense oligonucleotide preferably contains a RNA residue, a DNA residue, and/or a nucleotide analogue or equivalent, as will be further detailed herein below.

A preferred molecule of the invention comprises a nucleotide-based or nucleotide or an antisense oligonucleotide sequence of between 21 and 50 nucleotides or bases, more preferred between 21 and 40 nucleotides, more preferred between 21 and 30 nucleotides, such as 21 nucleotides, 22 nucleotides, 23 nucleotides, 24 nucleotides, 25 nucleotides, 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, 40 nucleotides, 41 nucleotides, 42 nucleotides, 43 nucleotides, 44 nucleotides, 45 nucleotides, 46 nucleotides, 47 nucleotides, 48 nucleotides, 49 nucleotides or 50 nucleotides.

A most preferred molecule of the invention comprises a nucleotide-based sequence of 25 nucleotides.

A molecule of the invention binds to a continuous stretch of or is complementary to or is antisense to at least a continuous stretch of at least 21 nucleotides within the nucleotide sequence SEQ ID NO:2.

In a certain embodiment, the invention provides a molecule comprising or consisting of an antisense nucleotide sequence selected from the antisense nucleotide sequences as depicted in Table 1, except SEQ ID NO:68. A molecule of the invention that is antisense to the sequence of SEQ ID NO 2, which is present in exon 45 of the DMD gene preferably comprises or consists of the antisense nucleotide sequence of SEQ ID NO 3; SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, SEQ ID NO 25, SEQ ID NO 26, SEQ ID NO 27, SEQ ID NO 28, SEQ ID NO 29, SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, SEQ ID NO 39, SEQ ID NO 40, SEQ ID NO 41, SEQ ID NO 42, SEQ ID NO 43, SEQ ID NO 44, SEQ ID NO 45, SEQ ID NO 46, SEQ ID NO 47, SEQ ID NO 48, SEQ ID NO 49, SEQ ID NO 50, SEQ ID NO 51, SEQ ID NO 52, SEQ ID NO 53, SEQ ID NO 54, SEQ ID NO 55, SEQ ID NO 56, SEQ ID NO 57, SEQ ID NO 58, SEQ ID NO 59, SEQ ID NO 60, SEQ ID NO 61, SEQ ID NO 62, SEQ ID NO 63, SEQ ID NO 64, SEQ ID NO 65, SEQ ID NO 66 and/or SEQ ID NO:67.

In a more preferred embodiment, the invention provides a molecule comprising or consisting of the antisense nucleotide sequence of SEQ ID NO 3; SEQ ID NO 4, SEQ ID NO 5, SEQ ID NO 6, SEQ ID NO 7 and/or SEQ ID NO 8.

In a most preferred embodiment, the invention provides a molecule comprising or consisting of the antisense nucleotide sequence of SEQ ID NO 3. It was found that this molecule is very efficient in modulating splicing of exon 45 of the DMD pre-mRNA in a muscle cell.

A nucleotide sequence of a molecule of the invention may contain a RNA residue, a DNA residue, a nucleotide analogue or equivalent as will be further detailed herein below. In addition, a molecule of the invention may encompass a functional equivalent of a molecule of the invention as defined herein.

It is preferred that a molecule of the invention comprises a or at least one residue that is modified to increase nuclease resistance, and/or to increase the affinity of the antisense nucleotide for the target sequence. Therefore, in a preferred embodiment, an antisense nucleotide sequence comprises a or at least one nucleotide analogue or equivalent, wherein a nucleotide analogue or equivalent is defined as a residue having a modified base, and/or a modified backbone, and/or a non-natural internucleoside linkage, or a combination of these modifications.

In a preferred embodiment, a nucleotide analogue or equivalent comprises a modified backbone. Examples of such backbones are provided by morpholino backbones, carbamate backbones, siloxane backbones, sulfide, sulfoxide and sulfone backbones, formacetyl and thioformacetyl backbones, methyleneformacetyl backbones, riboacetyl backbones, alkene containing backbones, sulfamate, sulfonate and sulfonamide backbones, methyleneimino and methylenehydrazino backbones, and amide backbones. Phosphorodiamidate morpholino oligomers are modified backbone oligonucleotides that have previously been investigated as antisense agents. Morpholino oligonucleotides have an uncharged backbone in which the deoxyribose sugar of DNA is replaced by a six membered ring and the phosphodiester linkage is replaced by a phosphorodiamidate linkage. Morpholino oligonucleotides are resistant to enzymatic degradation and appear to function as antisense agents by arresting translation or interfering with pre-mRNA splicing rather than by activating RNase H. Morpholino oligonucleotides have been successfully delivered to tissue culture cells by methods that physically disrupt the cell membrane, and one study comparing several of these methods found that scrape loading was the most efficient method of delivery; however, because the morpholino backbone is uncharged, cationic lipids are not effective mediators of morpholino oligonucleotide uptake in cells. A recent report demonstrated triplex formation by a morpholino oligonucleotide and, because of the non-ionic backbone, these studies showed that the morpholino oligonucleotide was capable of triplex formation in the absence of magnesium.

It is further preferred that the linkage between a residue in a backbone does not include a phosphorus atom, such as a linkage that is formed by short chain alkyl or cycloalkyl internucleoside linkages, mixed heteroatom and alkyl or cycloalkyl internucleoside linkages, or one or more short chain heteroatomic or heterocyclic internucleoside linkages.

A preferred nucleotide analogue or equivalent comprises a Peptide Nucleic Acid (PNA), having a modified polyamide backbone (Nielsen, et al. (1991) Science 254, 1497-1500). PNA-based molecules are true mimics of DNA molecules in terms of base-pair recognition. The backbone of the PNA is composed of *N*-(2-aminoethyl)-glycine units linked by peptide bonds, wherein the nucleobases are linked to the backbone by methylene carbonyl bonds. An alternative backbone comprises a one-carbon extended pyrrolidine PNA monomer (Govindaraju and Kumar (2005) Chem. Commun, 495-497). Since the backbone of a PNA molecule contains no charged phosphate groups, PNA-RNA hybrids are usually more stable than RNA-RNA or RNA-DNA hybrids, respectively (Egholm et al (1993) Nature 365, 566-568).

A further preferred backbone comprises a morpholino nucleotide analog or equivalent, in which the ribose or deoxyribose sugar is replaced by a 6-membered morpholino ring. A most preferred nucleotide analog or equivalent comprises a phosphorodiamidate morpholino oligomer (PMO), in which the ribose or deoxyribose sugar is replaced by a 6-membered morpholino ring, and the anionic phosphodiester linkage between adjacent morpholino rings is replaced by a non-ionic phosphorodiamidate linkage.

In yet a further embodiment, a nucleotide analogue or equivalent of the invention comprises a substitution of at least one of the non-bridging oxygens in the phosphodiester linkage. This modification slightly destabilizes base-pairing but adds significant resistance to nuclease degradation. A preferred nucleotide analogue or equivalent comprises phosphorothioate, chiral phosphorothioate, phosphorodithioate, phosphotriester, aminoalkylphosphotriester, H-phosphonate, methyl and other alkyl phosphonate including 3'-alkylene phosphonate, 5'-alkylene phosphonate and chiral phosphonate, phosphinate, phosphoramidate including 3'-amino phosphoramidate and aminoalkylphosphoramidate, thionophosphoramidate, thionoalkylphosphonate, thionoalkylphosphotriester, selenophosphate or boranophosphate.

A further preferred nucleotide analogue or equivalent of the invention comprises one or more sugar moieties that are mono- or disubstituted at the 2', 3' and/or 5' position such as a -OH; -F; substituted or unsubstituted, linear or branched lower (C1-C10) alkyl, alkenyl, alkynyl, alkaryl, allyl, aryl, or aralkyl, that may be interrupted by one or more heteroatoms; O-, S-, or N-alkyl; O-, S-, or N-alkenyl; O-, S-or N-alkynyl; O-, S-, or N-allyl; O-alkyl-O-alkyl, -methoxy, -aminopropoxy; aminoxy, methoxyethoxy; -dimethylaminooxyethoxy; and - dimethylaminoethoxyethoxy. The sugar moiety can be a pyranose or derivative thereof, or a deoxypyranose or derivative thereof, preferably a ribose or a derivative thereof, or deoxyribose or derivative thereof. Such preferred derivatized sugar moieties comprise Locked Nucleic Acid (LNA), in which the 2'-carbon atom is linked to the 3' or 4' carbon atom of the sugar ring thereby forming a bicyclic sugar moiety. A preferred LNA comprises 2'-*O*,4'-*C*-ethylene-bridged nucleic acid (Morita et al. 2001. Nucleic Acid Res Supplement No. 1: 241-242). These substitutions render the nucleotide analogue or equivalent RNase H and nuclease resistant and increase the affinity for the target RNA.

It is understood by a skilled person that it is not necessary for all positions in an antisense oligonucleotide to be modified uniformly. In addition, more than one of the aforementioned analogues or equivalents may be incorporated in a single antisense oligonucleotide or even at a single position within an antisense oligonucleotide. In certain embodiments, an antisense oligonucleotide of the invention has at least two different types of analogues or equivalents.

A preferred antisense oligonucleotide according to the invention comprises a 2'-*O* -alkyl phosphorothioate antisense oligonucleotide, such as 2'-*O*-methyl modified ribose (RNA), 2'-*O*-ethyl modified ribose, 2'-*O*-propyl modified ribose, and/or substituted derivatives of these modifications such as halogenated derivatives.

A most preferred antisense oligonucleotide according to the invention comprises a 2'-*O*-methyl phosphorothioate ribose.

A functional equivalent of a molecule of the invention may be defined as an oligonucleotide as defined herein wherein an activity of said functional equivalent is retained to at least some extent. Preferably, an activity of said functional equivalent is inducing exon 45 skipping and providing a functional dystrophin protein. Said activity of said functional equivalent is therefore preferably assessed by detection of exon 45 skipping and quantifying the amount of a functional dystrophin protein. A functional dystrophin is herein preferably defined as being a dystrophin able to bind actin and members of the DGC protein complex. The assessment of said activity of an oligonucleotide is preferably done by RT-PCR or by immunofluorescence or Western blot analysis. Said activity is preferably retained to at least some extent when it represents at least 50%, or at least 60%, or at least 70% or at least 80% or at least 90% or at least 95% or more of corresponding activity of said oligonucleotide the functional equivalent derives from. Throughout this application, when the word oligonucleotide is used it may be replaced by a functional equivalent thereof as defined herein.

It will also be understood by a skilled person that distinct antisense oligonucleotides can be combined for efficiently skipping of exon 45 of the human DMD pre-mRNA. In a preferred embodiment, a combination of at least two antisense oligonucleotides are used in a method of the invention, such as two distinct antisense oligonucleotides, three distinct antisense oligonucleotides, four distinct antisense oligonucleotides, or five distinct antisense oligonucleotides or even more. It is also encompassed by the present invention to combine several oligonucleotides or molecules as depicted in table 1 except SEQ ID NO:68.

An antisense oligonucleotide can be linked to a moiety that enhances uptake of the antisense oligonucleotide in cells, preferably myogenic cells or muscle cells. Examples of such moieties are cholesterols, carbohydrates, vitamins, biotin, lipids, phospholipids, cell-penetrating peptides including but not limited to antennapedia, TAT, transportan and positively charged amino acids such as oligoarginine, poly-arginine, oligolysine or polylysine, antigen-binding domains such as provided by an antibody, a Fab fragment of an antibody, or a single chain antigen binding domain such as a cameloid single domain antigen-binding domain.

A preferred antisense oligonucleotide comprises a peptide-linked PMO.

A preferred antisense oligonucleotide comprising one or more nucleotide analogs or equivalents of the invention modulates splicing in one or more muscle cells, including heart muscle cells, upon systemic delivery. In this respect, systemic delivery of an antisense oligonucleotide comprising a specific nucleotide analog or equivalent might result in targeting a subset of muscle cells, while an antisense oligonucleotide comprising a distinct nucleotide analog or equivalent might result in targeting of a different subset of muscle cells. Therefore, in one embodiment it is preferred to use a combination of antisense oligonucleotides comprising different nucleotide analogs or equivalents for modulating skipping of exon 45 of the human DMD pre-mRNA.

A cell can be provided with a molecule capable of interfering with essential sequences that result in highly efficient skipping of exon 45 of the human DMD pre-mRNA by plasmid-derived antisense oligonucleotide expression or viral expression provided by viral-based vector. Such a viral-based vector comprises an expression cassette that drives expression of an antisense molecule as defined herein. Preferred virus-based vectors include adenovirus- or adeno-associated virus-based vectors. Expression is preferably driven by a polymerase III promoter, such as a U1, a U6, or a U7 RNA promoter. A muscle or myogenic cell can be provided with a plasmid for antisense oligonucleotide expression by providing the plasmid in an aqueous solution. Alternatively, a plasmid can be provided by transfection using known transfection agentia such as, for example, LipofectAMINE™ 2000 (Invitrogen) or polyethyleneimine (PEI; ExGen500 (MBI Fermentas)), or derivatives thereof.

One preferred antisense oligonucleotide expression system is an adenovirus associated virus (AAV)-based vector. Single chain and double chain AAV-based vectors have been developed that can be used for prolonged expression of small antisense nucleotide sequences for highly efficient skipping of exon 45 of the DMD pre-mRNA.

A preferred AAV-based vector comprises an expression cassette that is driven by a polymerase III-promoter (Pol III). A preferred Pol III promoter is, for example, a U1, a U6, or a U7 RNA promoter.

The invention therefore also provides a viral-based vector, comprising a Pol III-promoter driven expression cassette for expression of one or more antisense sequences of the invention for inducing skipping of exon 45 of the human DMD pre-mRNA.

### Pharmaceutical composition

If required, a molecule or a vector expressing an antisense oligonucleotide of the invention can be incorporated into a pharmaceutically active mixture or composition by adding a pharmaceutically acceptable carrier.

Therefore, in a further aspect, the invention provides a composition, preferably a pharmaceutical composition comprising a molecule comprising an antisense oligonucleotide according to the invention, and/or a viral-based vector expressing the antisense sequence(s) according to the invention and a pharmaceutically acceptable carrier.

A preferred pharmaceutical composition comprises a molecule as defined herein and/or a vector as defined herein, and a pharmaceutical acceptable carrier or excipient, optionally combined with a molecule and/or a vector which is able to modulate skipping of exon 7, 44, 46, 51, 53, 59, 67 of the DMD pre-mRNA.

Preferred excipients include excipients capable of forming complexes, vesicles and/or liposomes that deliver such a molecule as defined herein, preferably an oligonucleotide complexed or trapped in a vesicle or liposome through a cell membrane. Many of these excipients are known in the art. Suitable excipients comprise polyethylenimine and derivatives, or similar cationic polymers, including polypropyleneimine or polyethylenimine copolymers (PECs) and derivatives, synthetic amphiphils, lipofectinTM, DOTAP and/or viral capsid proteins that are capable of self assembly into particles that can deliver such molecule, preferably an oligonucleotide as defined herein to a cell, preferably a muscle cell. Such excipients have been shown to efficiently deliver (oligonucleotide such as antisense) nucleic acids to a wide variety of cultured cells, including muscle cells. We obtained very good results using polyethylenimine (PEI, ExGen500, MBI Fermentas) as shown in the example. Their high transfection potential is combined with an excepted low to moderate toxicity in terms of overall cell survival. The ease of structural modification can be used to allow further modifications and the analysis of their further (*in vivo*) nucleic acid transfer characteristics and toxicity.

Lipofectin represents an example of a liposomal transfection agent. It consists of two lipid components, a cationic lipid N-[1-(2,3 dioleoyloxy)propyl]-N,N,N-trimethylammonium chloride (DOTMA) (cp. DOTAP which is the methylsulfate salt) and a neutral lipid dioleoylphosphatidylethanolamine (DOPE). The neutral component mediates the intracellular release. Another group of delivery systems are polymeric nanoparticles.

Polycations such like diethylaminoethylaminoethyl (DEAE)-dextran, which are well known as DNA transfection reagent can be combined with butylcyanoacrylate (PBCA) and hexylcyanoacrylate (PHCA) to formulate cationic nanoparticles that can deliver a molecule or a compound as defined herein, preferably an oligonucleotide across cell membranes into cells.

In addition to these common nanoparticle materials, the cationic peptide protamine offers an alternative approach to formulate a compound as defined herein, preferably an oligonucleotide as colloids. This colloidal nanoparticle system can form so called proticles, which can be prepared by a simple self-assembly process to package and mediate intracellular release of a compound as defined herein, preferably an oligonucleotide. The skilled person may select and adapt any of the above or other commercially available alternative excipients and delivery systems to package and deliver a compound as defined herein, preferably an oligonucleotide for use in the current invention to deliver said compound for the treatment of Duchenne Muscular Dystrophy in humans.

In addition, a compound as defined herein, preferably an oligonucleotide could be covalently or non-covalently linked to a targeting ligand specifically designed to facilitate the uptake in to the cell, cytoplasm and/or its nucleus. Such ligand could comprise (i) a compound (including but not limited to peptide(-like) structures) recognising cell, tissue or organ specific elements facilitating cellular uptake and/or (ii) a chemical compound able to facilitate the uptake in to cells and/or the intracellular release of an a compound as defined herein, preferably an oligonucleotide from vesicles, e.g. endosomes or lysosomes.

Therefore, in a preferred embodiment, a compound as defined herein, preferably an oligonucleotide are formulated in a medicament which is provided with at least an excipient and/or a targeting ligand for delivery and/or a delivery device of said compound to a cell and/or enhancing its intracellular delivery. Accordingly, the invention also encompasses a pharmaceutically acceptable composition comprising a compound as defined herein, preferably an oligonucleotide and further comprising at least one excipient and/or a targeting ligand for delivery and/or a delivery device of said compound to a cell and/or enhancing its intracellular delivery.
It is to be understood that a molecule or compound or oligonucleotide may not be formulated in one single composition or preparation. Depending on their identity, the skilled person will know which type of formulation is the most appropriate for each compound.

In a preferred embodiment, an in vitro concentration of a molecule or an oligonucleotide as defined herein, which is ranged between 0.1 nM and 1 µM is used. More preferably, the concentration used is ranged between 0.3 to 400 nM, even more preferably between 1 to 200 nM. molecule or an oligonucleotide as defined herein may be used at a dose which is ranged between 0.1 and 20 mg/kg, preferably 0.5 and 10 mg/kg. If several molecules or oligonucleotides are used, these concentrations may refer to the total concentration of oligonucleotides or the concentration of each oligonucleotide added. The ranges of concentration of oligonucleotide(s) as given above are preferred concentrations for *in vitro* or *ex vivo* uses. The skilled person will understand that depending on the oligonucleotide(s) used, the target cell to be treated, the gene target and its expression levels, the medium used and the transfection and incubation conditions, the concentration of oligonucleotide(s) used may further vary and may need to be optimised any further.

More preferably, a compound preferably an oligonucleotide and an adjunct compound to be used in the invention to prevent, treat DMD are synthetically produced and administered directly to a cell, a tissue, an organ and/or patients in formulated form in a pharmaceutically acceptable composition or preparation. The delivery of a pharmaceutical composition to the subject is preferably carried out by one or more parenteral injections, e.g. intravenous and/or subcutaneous and/or intramuscular and/or intrathecal and/or intraventricular administrations, preferably injections, at one or at multiple sites in the human body.

### Use

In yet a further aspect, the invention provides an antisense oligonucleotide or molecule according to the invention, and/or a viral-based vector that expresses one or more antisense sequences according to the invention and/or a pharmaceutical composition, for inducing and/or promoting splicing of the DMD pre-mRNA. for the treatment of DMD. The splicing is preferably modulated in a human myogenic cell or a muscle cell *in vitro*.

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that a molecule or a viral-based vector or a composition as defined herein may comprise additional component(s) than the ones specifically identified, said additional component(s) not altering the unique characteristic of the invention. In addition, reference to an element by the indefinite article "a" or "an" does not exclude the possibility that more than one of the element is present, unless the context clearly requires that there be one and only one of the elements. The indefinite article "a" or "an" thus usually means "at least one".

The following examples are offered for illustrative purposes only, and are not intended to limit the scope of the present invention in any way.

### Legends to the figure

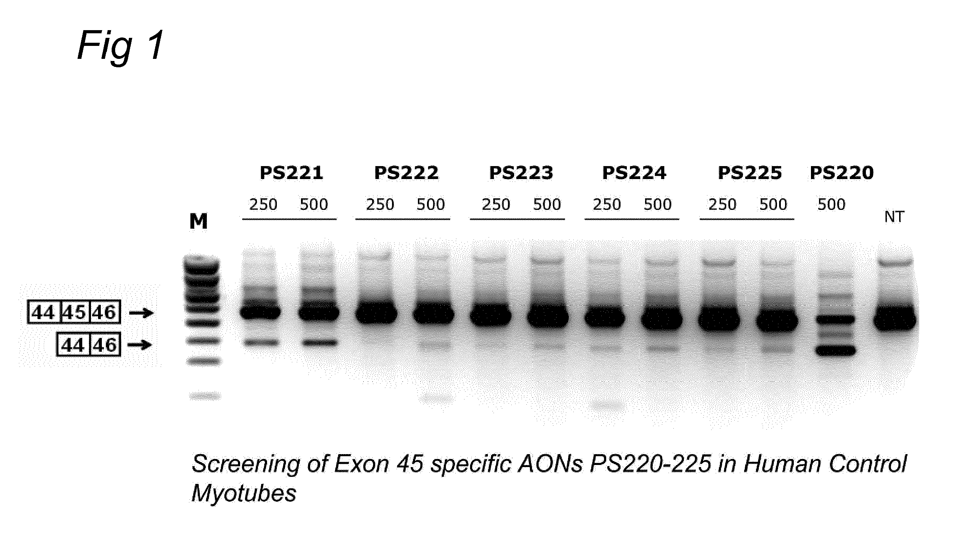

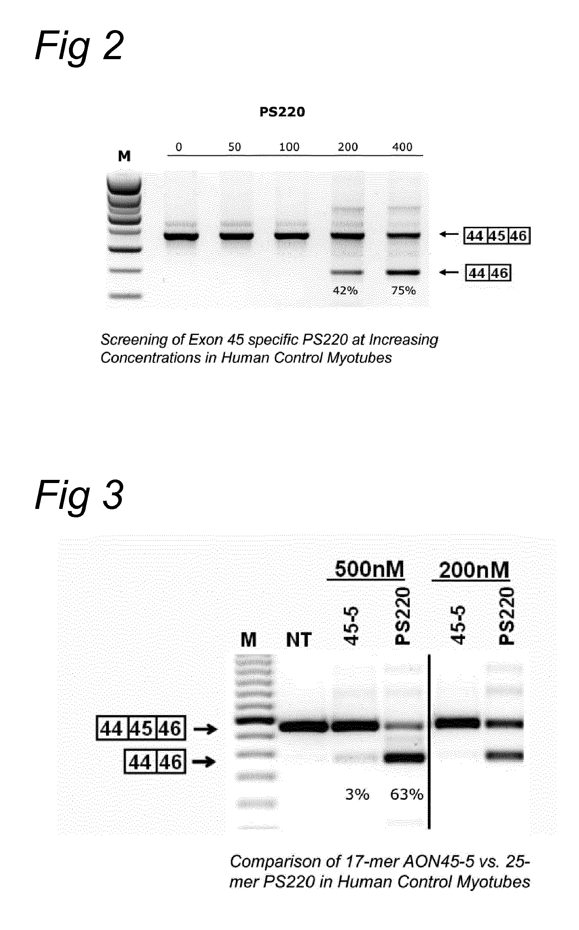

**Figure 1****.** In human control myotubes, a series of AONs (PS220 to PS225; SEQ ID NO: 3 to 8), all binding to a continuous stretch of at least 21 nucleotides within a specific sequence of exon 45 (i.e. SEQ ID NO:2), were tested at two different concentrations (200 and 500 nM). All six AONs were effective in inducing specific exon 45 skipping, as confirmed by sequence analysis (not shown). PS220 (SEQ ID NO:3) however, reproducibly induced highest levels of exon 45 skipping (see Fig. 2). (NT: non-treated cells, M: size marker).
**Figure 2****.** In human control myotubes, 25-mer PS220 (SEQ ID NO: 3) was tested at increasing concentration. Levels of exon 45 skipping of up to 75% (at 400 nM) were observed reproducibly, as assessed by Agilent LabChip Analysis.
**Figure 3****.** In human control myotubes, the efficiencies of a "short" 17-mer AON45-5 (SEQ ID NO:68) and its overlapping "long" 25-mer counterpart PS220 were directly compared at 200 nM and 500 nM. PS220 was markedly more efficient at both concentrations: 63% when compared to 3% obtained with 45-5. (NT: non-treated cells, M: size marker).

### Examples

### Examples 1 and 2

### Materials and methods

AON design was based on (partly) overlapping open secondary structures of the target exon RNA as predicted by the m-fold program (Zuker,M. (2003) Mfold web server for nucleic acid folding and hybridization prediction. Nucleic Acids Res., 31, 3406-3415), and on (partly) overlapping putative SR-protein binding sites as predicted by numerous software programs such as ESEfinder (Cartegni,L. et al.(2003) ESEfinder: A web resource to identify exonic splicing enhancers. Nucleic Acids Res, 31, 3568-71; Smith,P.J. et al. (2006) An increased specificity score matrix for the prediction of SF2/ASF-specific exonic splicing enhancers. Hum.Mol.Genet., 15, 2490-2508) that predicts binding sites for the four most abundant SR proteins (SF2/ASF, SC35, SRp40 and SRp55). AONs were synthesized by Prosensa Therapeutics B.V. (Leiden, Netherlands), and contain 2'-*O*-methyl RNA and full-length phosphorothioate (PS) backbones.

### Tissue culturing, transfection and RT-PCR analysis

Myotube cultures derived from a healthy individual ("human control") were obtained as described previously (Aartsma-Rus et al. Hum Mol Genet 2003; 12(8): 907-14). For the screening of AONs, myotube cultures were transfected with 0 to 500 nM of each AON. The transfection reagent polyethylenimine (PEI, ExGen500 MBI Fermentas) was used according to manufacturer's instructions, with 2 µl PEI per µg AON. Exon skipping efficiencies were determined by nested RT-PCR analysis using primers in the exons flanking exon 45. PCR fragments were isolated from agarose gels for sequence verification. For quantification, the PCR products were analyzed using the Agilent DNA 1000 LabChip Kit and the Agilent 2100 bioanalyzer (Agilent Technologies, USA).

### Results

A series of AONs targeting sequences within SEQ ID NO:2 within exon 45 were designed and tested in normal myotube cultures, by transfection and subsequent RT-PCR and sequence analysis of isolated RNA. PS220 (SEQ ID NO: 3) reproducibly induced highest levels of exon 45 skipping, when compared to PS221-PS225 (Figure 1). High levels of exon 45 skipping of up to 75% were already obtained at 400 nM PS220 (Figure 2). In a direct comparison, PS220 (a 25-mer) was reproducibly more efficient in inducing exon 45 skipping than its shorter 17-mer counterpart AON 45-5 (SEQ ID NO: 68; previously published as h45AON5 (Aartsma-Rus et al. Am J Hum Genet 2004;74: 83-92)), at both AON concentrations of 200 nM and 500 nM and with 63% versus 3% respectively at 500 nM (Figure 3). This result is probably due to the fact that the extended length of PS220, in fact completely overlapping AON 45-5, increases the free energy of the AON-target complex such that the efficiency of inducing exon 45 skipping is also increased.

**Table 1: AONs in exon 45**

| | | | |
|---|---|---|---|
| SEQ ID NO 3 (PS220) | UUUGCCGCUGCCCAAUGCCAUCCUG | SEQ ID NO 36 | GUUGCAUUCAAUGUUCUGACAACAG |
| SEQ ID NO 4 (PS221) | AUUCAAUGUUCUGACAACAGUUUGC | SEQ ID NO 37 | UUGCAUUCAAUGUUCUGACAACAGU |
| SEQ ID NO 5 (PS222) | CCAGUUGCAUUCAAUGUUCUGACAA | SEQ ID NO 38 | UGCAUUCAAUGUUCUGACAACAGUU |
| SEQ ID NO 6 (PS223) | CAGUUGCAUUCAAUGUUCUGAC | SEQ ID NO 39 | GCAUUCAAUGUUCUGACAACAGUUU |
| SEQ ID NO 7 (PS224) | AGUUGCAUUCAAUGUUCUGA | SEQ ID NO 40 | CAUUCAAUGUUCUGACAACAGUUUG |
| SEQ ID NO 8 (PS225) | GAUUGCUGAAUUAUUUCUUCC | SEQ ID NO 41 | AUUCAAUGUUCUGACAACAGUUUGC |
| SEQ ID NO 9 | GAUUGCUGAAUUAUUUCUUCCCCAG | SEQ ID NO 42 | UCAAUGUUCUGACAACAGUUUGCCG |
| SEQ ID NO 10 | AUUGCUGAAUUAUUUCUUCCCCAGU | SEQ ID NO 43 | CAAUGUUCUGACAACAGUUUGCCGC |
| SEQ ID NO 11 | UUGCUGAAUUAUUUCUUCCCCAGUU | SEQ ID NO 44 | AAUGUUCUGACAACAGUUUGCCGCU |
| SEQ ID NO 12 | UGCUGAAUUAUUUCUUCCCCAGUUG | SEQ ID NO 45 | AUGUUCUGACAACAGUUUGCCGCUG |
| SEQ ID NO 13 | GCUGAAUUAUUUCUUCCCCAGUUGC | SEQ ID NO 46 | UGUUCUGACAACAGUUUGCCGCUGC |
| SEQ ID NO 14 | CUGAAUUAUUUCUUCCCCAGUUGCA | SEQ ID NO 47 | GUUCUGACAACAGUUUGCCGCUGCC |
| SEQ ID NO 15 | UGAAUUAUUUCUUCCCCAGUUGCAU | SEQ ID NO 48 | UUCUGACAACAGUUUGCCGCUGCCC |
| SEQ ID NO 16 | GAAUUAUUUCUUCCCCAGUUGCAUU | SEQ ID NO 49 | UCUGACAACAGUUUGCCGCUGCCCA |
| SEQ ID NO 17 | AAUUAUUUCUUCCCCAGUUGCAUUC | SEQ ID NO 50 | CUGACAACAGUUUGCCGCUGCCCAA |
| SEQ ID NO 18 | AUUAUUUCUUCCCCAGUUGCAUUCA | SEQ ID NO 51 | UGACAACAGUUUGCCGCUGCCCAAU |
| SEQ ID NO 19 | UUAUUUCUUCCCCAGUUGCAUUCAA | SEQ ID NO 52 | GACAACAGUUUGCCGCUGCCCAAUG |
| SEQ ID NO 20 | UAUUUCUUCCCCAGUUGCAUUCAAU | SEQ ID NO 53 | ACAACAGUUUGCCGCUGCCCAAUGC |
| SEQ ID NO 21 | AUUUCUUCCCCAGUUGCAUUCAAUG | SEQ ID NO 54 | CAACAGUUUGCCGCUGCCCAAUGCC |
| SEQ ID NO 22 | UUUCUUCCCCAGUUGCAUUCAAUGU | SEQ ID NO 55 | AACAGUUUGCCGCUGCCCAAUGCCA |
| SEQ ID NO 23 | UUCUUCCCCAGUUGCAUUCAAUGUU | SEQ ID NO 56 | ACAGUUUGCCGCUGCCCAAUGCCAU |
| SEQ ID NO 24 | UCUUCCCCAGUUGCAUUCAAUGUUC | SEQ ID NO 57 | CAGUUUGCCGCUGCCCAAUGCCAUC |
| SEQ ID NO 25 | CUUCCCCAGUUGCAUUCAAUGUUCU | SEQ ID NO 58 | AGUUUGCCGCUGCCCAAUGCCAUCC |
| SEQ ID NO 26 | UUCCCCAGUUGCAUUCAAUGUUCUG | SEQ ID NO 59 | GUUUGCCGCUGCCCAAUGCCAUCCU |
| SEQ ID NO 27 | UCCCCAGUUGCAUUCAAUGUUCUGA | SEQ ID NO 60 | UUUGCCGCUGCCCAAUGCCAUCCUG |
| SEQ ID NO 28 | CCCCAGUUGCAUUCAAUGUUCUGAC | SEQ ID NO 61 | UUGCCGCUGCCCAAUGCCAUCCUGG |
| SEQ ID NO 29 | CCCAGUUGCAUUCAAUGUUCUGACA | SEQ ID NO 62 | UGCCGCUGCCCAAUGCCAUCCUGGA |
| SEQ ID NO 30 | CCAGUUGCAUUCAAUGUUCUGACAA | SEQ ID NO 63 | GCCGCUGCCCAAUGCCAUCCUGGAG |
| SEQ ID NO 31 | CAGUUGCAUUCAAUGUUCUGACAAC | SEQ ID NO 64 | CCGCUGCCCAAUGCCAUCCUGGAGU |
| SEQ ID NO 32 | AGUUGCAUUCAAUGUUCUGACAACA | SEQ ID NO 65 | CGCUGCCCAAUGCCAUCCUGGAGUU |
| SEQ ID NO 33 | UCC UGU AGA AUA CUG GCA UC | SEQ ID NO 66 | UGU UUU UGA GGA UUG CUG AA |
| SEQ ID NO 34 | | SEQ ID NO 67 | |
| SEQ ID NO 35 | | SEQ ID NO 68 (45-5) | GCCCAAUGCCAUCCUGG |

**Table 2 AONs in exons 51, 53, 7, 44, 46, 59, and 67**

| **DMD Gene Exon 51** | | | |
|---|---|---|---|
| SEQ ID NO 69 | AGAGCAGGUACCUCCAACAUCAAGG | SEQ ID NO 91 | UCAAGGAAGAUGGCAUUUCUAGUUU |
| SEQ ID NO 70 | GAGCAGGUACCUCCAACAUCAAGGA | SEQ ID NO 92 | UCAAGGAAGAUGGCAUUUCU |
| SEQ ID NO 71 | AGCAGGUACCUCCAACAUCAAGGAA | SEQ ID NO 93 | CAAGGAAGAUGGCAUUUCUAGUUUG |
| SEQ ID NO 72 | GCAGGUACCUCCAACAUCAAGGAAG | SEQ ID NO 94 | AAGGAAGAUGGCAUUUCUAGUUUGG |
| SEQ ID NO 73 | CAGGUACCUCCAACAUCAAGGAAGA | SEQ ID NO 95 | AGGAAGAUGGCAUUUCUAGUUUGGA |
| SEQ ID NO 74 | AGGUACCUCCAACAUCAAGGAAGAU | SEQ ID NO 96 | GGAAGAUGGCAUUUCUAGUUUGGAG |
| SEQ ID NO 75 | GGUACCUCCAACAUCAAGGAAGAUG | SEQ ID NO 97 | GAAGAUGGCAUUUCUAGUUUGGAGA |
| SEQ ID NO 76 | GUACCUCCAACAUCAAGGAAGAUGG | SEQ ID NO 98 | AAGAUGGCAUUUCUAGUUUGGAGAU |
| SEQ ID NO 77 | UACCUCCAACAUCAAGGAAGAUGGC | SEQ ID NO 99 | AGAUGGCAUUUCUAGUUUGGAGAUG |
| SEQ ID NO 78 | ACCUCCAACAUCAAGGAAGAUGGCA | SEQ ID NO 100 | GAUGGCAUUUCUAGUUUGGAGAUGG |
| SEQ ID NO 79 | CCUCCAACAUCAAGGAAGAUGGCAU | SEQ ID NO 101 | AUGGCAUUUCUAGUUUGGAGAUGGC |
| SEQ ID NO 80 | CUCCAACAUCAAGGAAGAUGGCAUU | SEQ ID NO 102 | UGGCAUUUCUAGUUUGGAGAUGGCA |
| SEQ ID NO 81 | CUCCAACAUCAAGGAAGAUGGCAUUUCUAG | SEQ ID NO 103 | GGCAUUUCUAGUUUGGAGAUGGCAG |
| SEQ ID NO 82 | UCCAACAUCAAGGAAGAUGGCAUUU | SEQ ID NO 104 | GCAUUUCUAGUUUGGAGAUGGCAGU |
| SEQ ID NO 83 | CCAACAUCAAGGAAGAUGGCAUUUC | SEQ ID NO 105 | CAUUUCUAGUUUGGAGAUGGCAGUU |
| SEQ ID NO 84 | CAACAUCAAGGAAGAUGGCAUUUCU | SEQ ID NO 106 | AUUUCUAGUUUGGAGAUGGCAGUUU |
| SEQ ID NO 85 | AACAUCAAGGAAGAUGGCAUUUCUA | SEQ ID NO 107 | UUUCUAGUUUGGAGAUGGCAGUUUC |
| SEQ ID NO 86 | ACAUCAAGGAAGAUGGCAUUUCUAG | SEQ ID NO 108 | UUCUAGUUUGGAGAUGGCAGUUUCC |
| SEQ ID NO 87 | ACAUCAAGGAAGAUGGCAUUUCUAGUUUGG | | |
| SEQ ID NO 88 | ACAUCAAGGAAGAUGGCAUUUCUAG | | |
| SEQ ID NO 89 | CAUCAAGGAAGAUGGCAUUUCUAGU | | |
| SEQ ID NO 90 | AUCAAGGAAGAUGGCAUUUCUAGUU | | |

| **DMD Gene Exon 53** | | | |
|---|---|---|---|
| SEQ ID NO 109 | CCAUUGUGUUGAAUCCUUUAACAUU | SEQ ID NO 116 | CAUUCAACUGUUGCCUCCGGUUCUGAAGGUG |
| SEQ ID NO 110 | CCAUUGUGUUGAAUCCUUUAAC | SEQ ID NO 117 | CUGAAGGUGUUCUUGUACUUCAUCC |
| SEQ ID NO 111 | AUUGUGUUGAAUCCUUUAAC | SEQ ID NO 118 | UGUAUAGGGACCCUCCUUCCAUGACUC |
| SEQ ID NO 112 | CCUGUCCUAAGACCUGCUCA | SEQ ID NO 119 | AUCCCACUGAUUCUGAAUUC |
| SEQ ID NO 113 | CUUUUGGAUUGCAUCUACUGUAUAG | SEQ ID NO 120 | UUGGCUCUGGCCUGUCCUAAGA |
| SEQ ID NO 114 | CAUUCAACUGUUGCCUCCGGUUCUG | SEQ ID NO 121 | AAGACCUGCUCAGCUUCUUCCUUAGCUUCCAGCCA |
| SEQ ID NO 115 | CUGUUGCCUCCGGUUCUGAAGGUG | | |

| **DMD Gene Exon 7** | | | |
|---|---|---|---|
| SEQ ID NO 122 | UGCAUGUUCCAGUCGUUGUGUGG | SEQ ID NO 124 | AUUUACCAACCUUCAGGAUCGAGUA |
| SEQ ID NO 123 | CACUAUUCCAGUCAAAUAGGUCUGG | SEQ ID NO 125 | GGCCUAAAACACAUACACAUA |

| **DMD Gene Exon 44** | | | |
|---|---|---|---|
| SEQ ID NO 126 | UCAGCUUCUGUUAGCCACUG | SEQ ID NO 151 | AGCUUCUGUUAGCCACUGAUUAAA |
| SEQ ID NO 127 | UUCAGCUUCUGUUAGCCACU | SEQ ID NO 152 | CAGCUUCUGUUAGCCACUGAUUAAA |
| SEQ ID NO 128 | UUCAGCUUCUGUUAGCCACUG | SEQ ID NO 153 | AGCUUCUGUUAGCCACUGAUUAAA |
| SEQ ID NO 129 | UCAGCUUCUGUUAGCCACUGA | SEQ ID NO 154 | AGCUUCUGUUAGCCACUGAU |
| SEQ ID NO 130 | UUCAGCUUCUGUUAGCCACUGA | SEQ ID NO 155 | GCUUCUGUUAGCCACUGAUU |
| SEQ ID NO 131 | UCAGCUUCUGUUAGCCACUGA | SEQ ID NO 156 | AGCUUCUGUUAGCCACUGAUU |
| SEQ ID NO 132 | UUCAGCUUCUGUUAGCCACUGA | SEQ ID NO 157 | GCUUCUGUUAGCCACUGAUUA |
| SEQ ID NO 133 | UCAGCUUCUGUUAGCCACUGAU | SEQ ID NO 158 | AGCUUCUGUUAGCCACUGAUUA |
| SEQ ID NO 134 | UUCAGCUUCUGUUAGCCACUGAU | SEQ ID NO 159 | GCUUCUGUUAGCCACUGAUUAA |
| SEQ ID NO 135 | UCAGCUUCUGUUAGCCACUGAUU | SEQ ID NO 160 | AGCUUCUGUUAGCCACUGAUUAA |
| SEQ ID NO 136 | UUCAGCUUCUGUUAGCCACUGAUU | SEQ ID NO 161 | GCUUCUGUUAGCCACUGAUUAAA |
| SEQ ID NO 137 | UCAGCUUCUGUUAGCCACUGAUUA | SEQ ID NO 162 | AGCUUCUGUUAGCCACUGAUUAAA |
| SEQ ID NO 138 | UUCAGCUUCUGUUAGCCACUGAUA | SEQ ID NO 163 | GCUUCUGUUAGCCACUGAUUAAA |
| SEQ ID NO 139 | UCAGCUUCUGUUAGCCACUGAUUAA | SEQ ID NO 164 | CCAUUUGUAUUUAGCAUGUUCCC |
| SEQ ID NO 140 | UUCAGCUUCUGUUAGCCACUGAUUAA | SEQ ID NO 165 | AGAUACCAUUUGUAUUUAGC |
| SEQ ID NO 141 | UCAGCUUCUGUUAGCCACUGAUUAAA | SEQ ID NO 166 | GCCAUUUCUCAACAGAUCU |
| SEQ ID NO 142 | UUCAGCUUCUGUUAGCCACUGAUUAAA | SEQ ID NO 167 | GCCAUUUCUCAACAGAUCUGUCA |
| SEQ ID NO 143 | CAGCUUCUGUUAGCCACUG | SEQ ID NO 168 | AUUCUCAGGAAUUUGUGUCUUUC |
| SEQ ID NO 144 | CAGCUUCUGUUAGCCACUGAU | SEQ ID NO 169 | UCUCAGGAAUUUGUGUCUUUC |
| SEQ ID NO 145 | AGCUUCUGUUAGCCACUGAUU | SEQ ID NO 170 | GUUCAGCUUCUGUUAGCC |
| SEQ ID NO 146 | CAGCUUCUGUUAGCCACUGAUU | SEQ ID NO 171 | CUGAUUAAAUAUCUUUAUAU C |
| SEQ ID NO 147 | AGCUUCUGUUAGCCACUGAUUA | SEQ ID NO 172 | GCCGCCAUUUCUCAACAG |
| SEQ ID NO 148 | CAGCUUCUGUUAGCCACUGAUUA | SEQ ID NO 173 | GUAUUUAGCAUGUUCCCA |
| SEQ ID NO 149 | AGCUUCUGUUAGCCACUGAUUAA | SEQ ID NO 174 | CAGGAAUUUGUGUCUUUC |
| SEQ ID NO 150 | CAGCUUCUGUUAGCCACUGAUUAA | | |

| **DMD Gene Exon 46** | | | |
|---|---|---|---|
| SEQ ID NO 175 | GCUUUUCUUUUAGUUGCUGCUCUUU | SEQ ID NO 203 | AGGUUCAAGUGGGAUACUAGCAAUG |
| SEQ ID NO 176 | CUUUUCUUUUAGUUGCUGCUCUUUU | SEQ ID NO 204 | GGUUCAAGUGGGAUACUAGCAAUGU |
| SEQ ID NO 177 | UUUUCUUUUAGUUGCUGCUCUUUUC | SEQ ID NO 205 | GUUCAAGUGGGAUACUAGCAAUGUU |
| SEQ ID NO 178 | UUUCUUUUAGUUGCUGCUCUUUUCC | SEQ ID NO 206 | UUCAAGUGGGAUACUAGCAAUGUUA |
| SEQ ID NO 179 | UUCUUUUAGUUGCUGCUCUUUUCCA | SEQ ID NO 207 | UCAAGUGGGAUACUAGCAAUGUUAU |
| SEQ ID NO 180 | UCUUUUAGUUGCUGCUCUUUUCCAG | SEQ ID NO 208 | CAAGUGGGAUACUAGCAAUGUUAUC |
| SEQ ID NO 181 | CUUUUAGUUGCUGCUCUUUUCCAGG | SEQ ID NO 209 | AAGUGGGAUACUAGCAAUGUUAUCU |
| SEQ ID NO 182 | UUUUAGUUGCUGCUCUUUUCCAGGU | SEQ ID NO 210 | AGUGGGAUACUAGCAAUGUUAUCUG |
| SEQ ID NO 183 | UUUAGUUGCUGCUCUUUUCCAGGUU | SEQ ID NO 211 | GUGGGAUACUAGCAAUGUUAUCUGC |
| SEQ ID NO 184 | UUAGUUGCUGCUCUUUUCCAGGUUC | SEQ ID NO 212 | UGGGAUACUAGCAAUGUUAUCUGCU |
| SEQ ID NO 185 | UAGUUGCUGCUCUUUUCCAGGUUCA | SEQ ID NO 213 | GGGAUACUAGCAAUGUUAUCUGCUU |
| SEQ ID NO 186 | AGUUGCUGCUCUUUUCCAGGUUCAA | SEQ ID NO 214 | GGAUACUAGCAAUGUUAUCUGCUUC |
| SEQ ID NO 187 | GUUGCUGCUCUUUUCCAGGUUCAAG | SEQ ID NO 215 | GAUACUAGCAAUGUUAUCUGCUUCC |
| SEQ ID NO 188 | UUGCUGCUCUUUUCCAGGUUCAAGU | SEQ ID NO 216 | AUACUAGCAAUGUUAUCUGCUUCCU |
| SEQ ID NO 189 | UGCUGCUCUUUUCCAGGUUCAAGUG | SEQ ID NO 217 | UACUAGCAAUGUUAUCUGCUUCCUC |
| SEQ ID NO 190 | GCUGCUCUUUUCCAGGUUCAAGUGG | SEQ ID NO 218 | ACUAGCAAUGUUAUCUGCUUCCUCC |
| SEQ ID NO 191 | CUGCUCUUUUCCAGGUUCAAGUGGG | SEQ ID NO 219 | CUAGCAAUGUUAUCUGCUUCCUCCA |
| SEQ ID NO 192 | UGCUCUUUUCCAGGUUCAAGUGGGA | SEQ ID NO 220 | UAGCAAUGUUAUCUGCUUCCUCCAA |
| SEQ ID NO 193 | GCUCUUUUCCAGGUUCAAGUGGGAC | SEQ ID NO 221 | AGCAAUGUUAUCUGCUUCCUCCAAC |
| SEQ ID NO 194 | CUCUUUUCCAGGUUCAAGUGGGAUA | SEQ ID NO 222 | GCAAUGUUAUCUGCUUCCUCCAACC |
| SEQ ID NO 195 | UCUUUUCCAGGUUCAAGUGGGAUAC | SEQ ID NO 223 | CAAUGUUAUCUGCUUCCUCCAACCA |
| SEQ ID NO 196 | CUUUUCCAGGUUCAAGUGGGAUACU | SEQ ID NO 224 | AAUGUUAUCUGCUUCCUCCAACCAU |
| SEQ ID NO 197 | UUUUCCAGGUUCAAGUGGGAUACUA | SEQ ID NO 225 | AUGUUAUCUGCUUCCUCCAACCAUA |
| SEQ ID NO 198 | UUUCCAGGUUCAAGUGGGAUACUAG | SEQ ID NO 226 | UGUUAUCUGCUUCCUCCAACCAUAA |
| SEQ ID NO 199 | UUCCAGGUUCAAGUGGGAUACUAGC | SEQ ID NO 227 | GUUAUCUGCUUCCUCCAACCAUAAA |
| SEQ ID NO 200 | UCCAGGUUCAAGUGGGAUACUAGCA | SEQ ID NO 228 | GCUGCUCUUUUCCAGGUUC |
| SEQ ID NO 201 | CCAGGUUCAAGUGGGAUACUAGCAA | SEQ ID NO 229 | UCUUUUCCAGGUUCAAGUGG |
| SEQ ID NO 202 | CAGGUUCAAGUGGGAUACUAGCAAU | SEQ ID NO 230 | AGGUUCAAGUGGGAUACUA |

| **DMD Gene Exon 59** | | | |
|---|---|---|---|
| SEQ ID NO 231 | CAAUUUUUCCCACUCAGUAUU | SEQ ID NO 233 | UCCUCAGGAGGCAGCUCUAAAU |
| SEQ ID NO 232 | UUGAAGUUCCUGGAGUCUU | | |

| **DMD Gene Exon 67** | | | |
|---|---|---|---|
| SEQ ID NO 234 | GCGCUGGUCACAAAAUCCUGUUGAAC | SEQ ID NO 236 | GGUGAAUAACUUACAAAUUUGGAAGC |
| SEQ ID NO 235 | CACUUGCUUGAAAAGGUCUACAAAGGA | | |
| | | | |

## Claims

1. An antisense oligonucleotide, whereby said antisense oligonucleotide is able to bind to a stretch of at least 21 nucleotides within the following nucleotide sequence within exon 45 of the dystrophin pre-mRNA: and wherein said oligonucleotide has a length of at least 21 nucleotides and wherein said antisense oligonucleotide does not consist of 5'- AACAGTTTGCCGCTGCCCAATGCCA - 3', 5'- CTGACAACAGTTTGCCGCTGCCCAA -3', 5' - GTTGCATTCAATGTTCTGACAACAG -3', 5' GCTGAATTATTTCTTCCCCAGTTGC - 3' or 5'- ATTATTTCTTCCCCAGTTGCATTCA -3'.

2. An antisense oligonucleotide according to claim 1, wherein the length of said oligonucleotide is 21 to 50 nucleotides.

3. An antisense oligonucleotide according to claim 1 or 2, wherein the base sequence of said oligonucleotide comprises or consists of one of the base sequences selected from:
5' AUUCAAUGUUCUGACAACAGUUUGC 3' (SEQ ID NO:4),
5' CCAGUUGCAUUCAAUGUUCUGACAA 3' (SEQ ID NO: 5),
5' CAGUUGCAUUCAAUGUUCUGAC 3' (SEQ ID NO: 6) and
5' GAUUGCUGAAUUAUUUCUUCC 3' (SEQ ID NO: 8)

4. An antisense oligonucleotide according to any one of claim 1 to 3, wherein at least one of the nucleotides is a nucleotide analogue.

5. An antisense oligonucleotide according to any one of claims 1-4, comprising one or more RNA residues and/or one or more DNA residues.

6. An antisense oligonucleotide according to claim 4, wherein the nucleotide analogue is defined as a nucleotide having a modified base, and/or a modified sugar moiety and/or a modified internucleoside linkage.

7. An antisense oligonucleotide according to claim 6, wherein the nucleotide analogue has a modified base and/or a modified internucleoside linkage.

8. An antisense oligonucleotide according to any one of claims 4-7 wherein the nucleotide analogue comprises one or more sugar moieties that are mono- or di-substituted at the 2', 3' and/or 5' position.

9. An antisense oligonucleotide according to any of claims 1 to 8, comprising a 2'-*O* substituted phosphorothioate antisense oligonucleotide.

10. An antisense oligonucleotide according to claim 9, comprising a 2'-*O* methyl phosphorothioate ribose.

11. An antisense oligonucleotide according to any one of claims 1-10, comprising a phosphorothioate antisense oligonucleotide, wherein the sugar moieties are each 2'-*O* methyl substituted and comprising one of the base sequences:
5' AUUCAAUGUUCUGACAACAGUUUGC 3' (SEQ ID NO:4),
5' CCAGUUGCAUUCAAUGUUCUGACAA 3' (SEQ ID NO: 5),
5' CAGUUGCAUUCAAUGUUCUGAC 3' (SEQ ID NO: 6) or
5' GAUUGCUGAAUUAUUUCUUCC 3' (SEQ ID NO: 8)

12. An antisense oligonucleotide which is a phosphorothioate antisense oligonucleotide wherein the sugar moieties are 2'-*O*-methyl substituted and having one of the base sequences:
5' AUUCAAUGUUCUGACAACAGUUUGC 3' (SEQ ID NO:4),
5' CCAGUUGCAUUCAAUGUUCUGACAA 3' (SEQ ID NO: 5),
5' CAGUUGCAUUCAAUGUUCUGAC 3' (SEQ ID NO: 6) or
5' GAUUGCUGAAUUAUUUCUUCC 3' (SEQ ID NO: 8)

13. An antisense oligonucleotide according to any one of claims 7-9, wherein the modified internucleoside linkage is selected from the group consisting of a morpholino backbone, a carbamate backbone, a siloxane backbone, a sulfide backbone, a sulfoxide backbone, a sulfone backbone, a formacetyl backbone, a thioformacetyl backbone, a methyleneformacetyl backbone, a riboacetyl backbone, an alkene containing backbone, a sulfamate backbone, a sulfonate backbone, a sulfonamide backbone, a methyleneimino backbone, a methylenehydrazino backbone and an amide backbone.

14. An antisense oligonucleotide according to any one of claims 1-10 or 13, comprising a phosphorodiamidate morpholino oligomer (PMO), peptide nucleic acid (PNA), and/or locked nucleic acid (LNA).

15. A viral-based vector, comprising an expression cassette that drives expression of the antisense oligonucleotide as defined in claim 1, 2 or 3.

16. A pharmaceutical composition comprising an antisense oligonucleotide as defined in any one of claims 1-14 and/or the vector of claim 15, a pharmaceutical acceptable carrier.

17. A pharmaceutical composition according to claim 16, further comprising an antisense oligonucleotide which is able to induce or promote skipping of exon 7, 44, 46, 51, 53, 59 or 67 (as depicted in table 2) in the dystrophin pre-mRNA of a patient or a viral-based vector comprising an expression cassette that drives expression of said antisense, wherein said composition is able to exclude at least the two targeted exons from the dystrophin pre-mRNA, preferably said composition is able to exclude the entire region in between said two targeted exons in said pre-mRNA.

18. The antisense oligonucleotide as defined in any one of claims 1-14, the vector of claim 15, or the pharmaceutical composition of claim 16, for inducing or promoting exon 45 skipping for use in treating a DMD or a BMD patient.

19. The pharmaceutical composition of claim 17 for skipping at least two targeted exons from the dystrophin pre-mRNA, preferably for skipping the entire region in between said two targeted exons in said pre-mRNA for use in treating a DMD or a BMD patient.

20. An in vitro method for inducing or promoting skipping of exon 45 of dystrophin pre- mRNA in an isolated cell of said patient, the method comprising providing said cell with an antisense oligonucleotide as defined in any one of claims 1-14, the vector of claim 15, or the pharmaceutical composition of claim 16.

## Patentansprüche

1. Antisense-Oligonucleotid, wobei das Antisense-Oligonucleotid in der Lage ist, an eine Kette von mindestens 21 Nucleotiden innerhalb der folgenden Nucleotidsequenz innerhalb Exon 45 der Dystrophin-Prä- mRNA zu binden: und wobei das Oligonucleotid eine Länge von mindestens 21 Nucleotiden aufweist und wobei das Antisense-Oligonucleotid nicht aus 5'-AACAGTTTGCCGCTGCCAATGCCA- 3', 5'-CTGACAACAGTTTGCCGCTGCCCAA-3', 5'-GTTGCATTCAATGTTCTGACAACAG-3', 5'-GCTGAATTATTTCTTCCCCAGTTGC-3' oder 5'-ATTATTTCTTCCCCAGTTGCATTCA-3' besteht.

2. Antisense-Oligonucleotid nach Anspruch 1, wobei die Länge des Oligonucleotids 21 bis 50 Nucleotide beträgt.

3. Antisense-Oligonucleotid nach Anspruch 1 oder 2, wobei die Basensequenz des Oligonucleotids eine der Basensequenzen umfasst oder daraus besteht, ausgewählt aus:
5'-AUUCAAUGUUCUGACAACAGUUUGC-3' (SEQ.-ID Nr. 4),
5'-CCAGUUGCAUUCAAUGUUCUGACAA-3' (SEQ.-ID Nr. 5),
5'-CAGUUGCAUUCAAUGUUCUGAC-3' (SEQ.-ID Nr. 6) und
5'-GAUUGCUGAAUUAUUUCUUCC-3' (SEQ.-ID Nr. 8).

4. Antisense-Oligonucleotid nach einem der Ansprüche 1 bis 3, wobei mindestens eines der Nucleotide ein Nucleotid-Analogon ist.

5. Antisense-Oligonucleotid nach einem der Ansprüche 1 bis 4, umfassend einen oder mehrere RNA-Reste und/oder einen oder mehrere DNA-Reste.

6. Antisense-Oligonucleotid nach Anspruch 4, wobei das Nucleotid-Analogon als ein Nucleotid definiert ist, welches eine modifizierte Base und/oder eine modifizierte Zuckereinheit und/oder eine modifizierte Internucleosid-Bindung aufweist.

7. Antisense-Oligonucleotid nach Anspruch 6, wobei das Nucleotid-Analogon eine modifizierte Base und/oder eine modifizierte Internucleosid-Bindung aufweist.

8. Antisense-Oligonucleotid nach einem der Ansprüche 4 bis 7, wobei das Nucleotid-Analogon eine oder mehrere Zuckereinheiten umfasst, die an der 2'-, 3'- und/oder 5'-Position mono- oder disubstituiert sind.

9. Antisense-Oligonucleotid nach einem der Ansprüche 1 bis 8, umfassend ein 2'-*O*-substituiertes Phosphothioat-Antisense-Oligonucleotid.

10. Antisense-Oligonucleotid nach Anspruch 9, umfassend eine 2'-*O*-Methylphosphothioat-Ribose.

11. Antisense-Oligonucleotid nach einem der Ansprüche 1 bis 10, umfassend ein Phosphothioat-Antisense-Oligonucleotid, wobei die Zuckereinheiten jeweils 2'-*O*-methylsubstituiert sind, und umfassend eine der Basensequenzen:
5'-AUUCAAUGUUCUGACAACAGUUUGC-3' (SEQ.-ID Nr. 4),
5'-CCAGUUGCAUUCAAUGUUCUGACAA-3' (SEQ.-ID Nr. 5),
5'-CAGUUGCAUUCAAUGUUCUGAC-3' (SEQ.-ID Nr. 6) oder
5'-GAUUGCUGAAUUAUUUCUUCC-3' (SEQ.-ID Nr. 8).

12. Antisense-Oligonucleotid welches ein Phosphothioat-Antisense-Oligonucleotid ist, wobei die Zuckereinheiten 2'-*O*-methylsubstituiert sind, und aufweisend eine der Basensequenzen:
5'-AUUCAAUGUUCUGACAACAGUUUGC-3' (SEQ.-ID Nr. 4),
5'-CCAGUUGCAUUCAAUGUUCUGACAA-3' (SEQ.-ID Nr. 5),
5'-CAGUUGCAUUCAAUGUUCUGAC-3' (SEQ.-ID Nr. 6) oder
5'-GAUUGCUGAAUUAUUUCUUCC-3' (SEQ.-ID Nr. 8).

13. Antisense-Oligonucleotid nach einem der Ansprüche 7 bis 9, wobei die modifizierte Internucleosid-Bindung aus der Gruppe ausgewählt ist, bestehend aus einem Morpholino-Grundgerüst, einem Carbamat-Grundgerüst, einem Siloxan-Grundgerüst, einem Sulfide-Grundgerüst, einem Sulfoxide-Grundgerüst, einem Sulfone-Grundgerüst, einem Formacetyl-Grundgerüst, einem Thioformacetyl-Grundgerüst, einem Methylenformacetyl-Grundgerüst, einem Riboacetyl-Grundgerüst, einem Alken enthaltenden Grundgerüst, einem Sulfamat-Grundgerüst, einem Sulfonat-Grundgerüst, einem Sulfonamid-Grundgerüst, einem Methylenimino-Grundgerüst, einem Methylenhydrazino-Grundgerüst und einem Amid-Grundgerüst.

14. Antisense-Oligonucleotid nach einem der Ansprüche 1 bis 10 oder 13, umfassend ein Phosphodiamidat-Morpholino-Oligomer (PMO), Peptid-Nucleinsäure (PNA) und/oder blockierte Nucleinsäure (Locked Nucleic Acid, LNA).

15. Vektor auf Virusbasis, umfassend eine Expressionskassette, welche die Expression des Antisense-Oligonucleotids nach Anspruch 1, 2 oder 3 betreibt.

16. Pharmazeutische Zusammensetzung, umfassend ein Antisense-Oligonucleotid nach einem der Ansprüche 1 bis 14 und/oder den Vektor nach Anspruch 15, einen pharmazeutisch unbedenklichen Träger.

17. Pharmazeutische Zusammensetzung nach Anspruch 16, ferner umfassend ein Antisense-Oligonucleotid, welches in der Lage ist, ein Überspringen von Exon 7, 44, 46, 51, 53, 59 oder 67 (wie in Tabelle 2 dargestellt) in der Dystrophin-Prä-mRNA eines Patienten oder einem Vektor auf Virusbasis, der eine Expressionskassette umfasst, welche die Expression des Antisense-Oligonucleotids betreibt, zu induzieren oder zu fördern, wobei die Zusammensetzung in der Lage ist, mindestens die zwei Ziel-Exone aus der Dystrophin-Prä-mRNA auszuschließen, wobei die Zusammensetzung vorzugsweise in der Lage ist, die gesamte Region zwischen den zwei Ziel-Exonen in der Prä-mRNA auszuschließen.

18. Antisense-Oligonucleotid nach einem der Ansprüche 1 bis 14, Vektor nach Anspruch 15 oder pharmazeutische Zusammensetzung nach Anspruch 16 zum Induzieren oder Fördern des Überspringens von Exon 45 zur Verwendung bei der Behandlung eines DMD- oder BMD-Patienten.

19. Pharmazeutische Zusammensetzung nach Anspruch 17 zum Überspringen von mindestens zwei Ziel-Exonen aus der Dystrophin-Prä-mRNA, vorzugsweise zum Überspringen der gesamten Region zwischen den zwei Ziel-Exonen in der Prä-mRNA, zur Verwendung bei der Behandlung eines DMD- oder BMD-Patienten.

20. In-vitro-Verfahren zum Induzieren oder Fördern des Überspringens von Exon 45 von Dystrophin-Prä-mRNA in einer isolierten Zelle des Patienten, wobei das Verfahren Versehen der Zelle mit einem Antisense-Oligonucleotid nach einem der Ansprüche 1 bis 14, dem Vektor nach Anspruch 15 oder der pharmazeutischen Zusammensetzung nach Anspruch 16 umfasst.

## Revendications

1. Oligonucléotide antisens, selon lequel ledit oligonucléotide antisens est capable de se lier à un segment d'au moins 21 nucléotides au sein de la séquence nucléotidique suivante au sein de l'exon 45 du pré-ARNm de la dystrophine : et dans lequel ledit oligonucléotide possède une longueur d'au moins 21 nucléotides et dans lequel ledit oligonucléotide antisens ne consiste pas en 5' - AACAGTTTGCCGCTGCCCAATGCCA -3', 5'- CTGACAACAGTTTGCCGCTGCCCAA -3', 5'- GTTGCATTCAATGTTCTGACAACAG -3', 5'- GCTGAATTATTTCTTCCCCAGTTGC -3' ou 5'- ATTATTTCTTCCCCAGTTGCATTCA -3'.

2. Oligonucléotide antisens selon la revendication 1, dans lequel la longueur dudit oligonucléotide est comprise entre 21 et 50 nucléotides.

3. Oligonucléotide antisens selon la revendication 1 ou la revendication 2, dans lequel la séquence de base dudit oligonucléotide comprend ou consiste en une des séquences de base choisie parmi :
5' AUUCAAUGUUCUGACAACAGUUUGC 3' (SEQ ID NO:4),
5' CCAGUUGCAUUCAAUGUUCUGACAA 3' (SEQ ID NO:5),
5' CAGUUGCAUUCAAUGUUCUGAC 3' (SEQ ID NO:6), et
5' GAUUGCUGAAUUAUUUCUUCC 3' (SEQ ID NO:8).

4. Oligonucléotide antisens selon l'une quelconque des revendications 1 à 3, dans lequel au moins un des nucléotides est un analogue de nucléotide.

5. Oligonucléotide antisens selon l'une quelconque des revendications 1 à 4, comprenant un ou plusieurs résidus d'ARN et/ou un ou plusieurs résidus d'ADN.

6. Oligonucléotide antisens selon la revendication 4, dans lequel l'analogue de nucléotide est défini en tant qu'un nucléotide possédant une base modifiée, et/ou une partie glucidique modifiée et/ou une liaison internucléosidique modifiée.

7. Oligonucléotide antisens selon la revendication 6, dans lequel l'analogue de nucléotide possède une base modifiée et/ou une liaison internucléosidique modifiée.

8. Oligonucléotide antisens selon l'une quelconque des revendications 4 à 7, dans lequel l'analogue de nucléotide comprend une ou plusieurs parties glucidiques qui sont mono- ou di-substituées en position 2', 3' et/ou 5'.

9. Oligonucléotide antisens selon l'une quelconque des revendications 1 à 8, comprenant un oligonucléotide antisens phosphorothioate substitué en 2'-0.

10. Oligonucléotide antisens selon la revendication 9, comprenant un 2'-0 méthylphosphorothioate ribose.

11. Oligonucléotide antisens selon l'une quelconque des revendications 1 à 10, comprenant un oligonucléotide antisens phosphorothioate, dans lequel les parties glucidiques sont chacune substituées par un groupe 2'-0 méthyle et comprenant une des séquences de base :
5' AUUCAAUGUUCUGACAACAGUUUGC 3' (SEQ ID NO:4),
5' CCAGUUGCAUUCAAUGUUCUGACAA 3' (SEQ ID NO:5),
5' CAGUUGCAUUCAAUGUUCUGAC 3' (SEQ ID NO:6), ou
5' GAUUGCUGAAUUAUUUCUUCC 3' (SEQ ID NO:8).

12. Oligonucléotide antisens qui est un oligonucléotide antisens phosphorothioate, dans lequel les parties glucidiques sont substituées par un groupe 2'-0 méthyle et possédant une des séquences de base :
5' AUUCAAUGUUCUGACAACAGUUUGC 3' (SEQ ID NO:4),
5' CCAGUUGCAUUCAAUGUUCUGACAA 3' (SEQ ID NO:5),
5' CAGUUGCAUUCAAUGUUCUGAC 3' (SEQ ID NO:6), ou
5' GAUUGCUGAAUUAUUUCUUCC 3' (SEQ ID NO:8).

13. Oligonucléotide antisens selon l'une quelconque des revendications 7 à 9, dans lequel la liaison internucléosidique modifiée est choisie parmi le groupe consistant en un squelette morpholino, un squelette carbamate, un squelette siloxane, un squelette sulfide, un squelette sulfoxyde, un squelette sulfone, un squelette formacétyle, un squelette thioformacétyle, un squelette méthylèneformacétyle, un squelette riboacétyle, un squelette contenant un alcène, un squelette sulfamate, un squelette sulfonate, un squelette sulfonamide, un squelette méthylèneimino, un squelette méthylenehydrazino et un squelette amide.

14. Oligonucléotide antisens selon l'une quelconque des revendications 1 à 10 ou 13, comprenant un oligomère morpholino phosphorodiamidate (PMO), un acide nucléique peptidique (PNA), et/ou un acide nucléique bloqué (LNA).

15. Vecteur à base virale, comprenant une cassette d'expression qui conduit l'expression de l'oligonucléotide antisens tel défini selon les revendications 1, 2 ou 3.

16. Composition pharmaceutique comprenant un oligonucléotide antisens tel que défini selon l'une quelconque des revendications 1 à 14 et/ou le vecteur selon la revendication 15, un support pharmaceutiquement acceptable.

17. Composition pharmaceutique selon la revendication 16, comprenant en outre un oligonucléotide antisens qui est capable d'induire ou de promouvoir l'omission de l'exon 7, 44, 46, 51, 53, 59 ou 67 (tel que décrit dans le tableau 2) dans le pré-ARNm de la dystrophine d'un patient ou d'un vecteur à base virale comprenant une cassette d'expression qui conduit l'expression dudit antisens, dans laquelle ladite composition est capable d'exclure au moins les deux exons ciblés à partir du pré-ARNm de la dystrophine, de préférence ladite composition est capable d'exclure l'entière région entre lesdits deux exons ciblés dans ledit pré-ARNm.

18. Oligonucléotide antisens selon l'une quelconque des revendications 1 à 14, le vecteur selon la revendication 15, ou la composition pharmaceutique selon la revendication 16, pour induire ou promouvoir l'omission de l'exon 45 destiné à une utilisation dans le traitement d'un patient DMD ou BMD.

19. Composition pharmaceutique selon la revendication 17 pour omettre au moins deux exons ciblés du pré-ARNm de la dystrophine, de préférence pour omettre l'entière région entre lesdits deux exons ciblés dans ledit pré-ARNm destinée à une utilisation dans le traitement d'un patient DMD ou BMD.

20. Procédé in vitro permettant d'induire ou de promouvoir l'omission de l'exon 45 du pré-ARNm de la dystrophine dans une cellule isolée dudit patient, le procédé comprenant la fourniture de ladite cellule avec un oligonucléotide antisens tel que défini selon l'une quelconque des revendications 1 à 14, le vecteur selon la revendication 15, ou la composition pharmaceutique selon la revendication 16.
